(19) Europäisches Patentamt
European Patent Office
Office européen des brevets

(11) EP 1 486 562 A1

(12) **EUROPEAN PATENT APPLICATION**
published in accordance with Art. 158(3) EPC

(43) Date of publication:
**15.12.2004 Bulletin 2004/51**

(21) Application number: **03703034.3**

(22) Date of filing: **23.01.2003**

(51) Int Cl.[7]: **C12N 15/09**, C12Q 1/68, G01N 33/15, G01N 33/50

(86) International application number:
**PCT/JP2003/000600**

(87) International publication number:
**WO 2003/072778 (04.09.2003 Gazette 2003/36)**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **27.02.2002 JP 2002052310**

(71) Applicants:
- **Genox Research, Inc.**
  **Ibaraki 300-2635 (JP)**
- **Japan as represented by General Director of Agency of National Center for Child Health and Development**
  **Tokyo 157-8535 (JP)**

(72) Inventors:
- **NAGATA, Naoko,**
  **c/o Teikyo Univ. Biotech Center**
  **Kawasaki-shi, Kanagawa 216-0001 (JP)**
- **OSHIDA, Tadahiro,**
  **c/o Teikyo Univ. Biotech Center**
  **Kawasaki-shi, Kanagawa 216-0001 (JP)**
- **SUGITA, Yuji, c/o Teikyo Univ. Biotech Center**
  **Kawasaki-shi, Kanagawa 216-0001 (JP)**
- **KUBO, Masato, c/o Science Univ. of Tokyo**
  **Noda-shi, C (JP)**
- **SAITO, Hirohisa, c/o National Research Institute**
  **Tokyo 154-8567 (JP)**

(74) Representative: **Grünecker, Kinkeldey,**
**Stockmair & Schwanhäusser Anwaltssozietät**
**Maximilianstrasse 58**
**80538 München (DE)**

(54) **METHOD OF EXAMINING ALLERGIC DISEASE**

(57) SOCS3 gene is identified as a gene whose expression level in T cells of patients with an atopic disorder is significantly higher than that of normal healthy subjects. The present inventors found that this gene can be used in testing for allergic diseases and in screening for therapeutic agents. The present invention enables screening for compounds useful in the treatment of allergic diseases.

EP 1 486 562 A1

Printed by Jouve, 75001 PARIS (FR)

SEQUENCE LISTING

<110> Genox Research, Inc.

<120> METHOD OF TESTING FOR ALLERGIC DISEASE

<130> G1-A0204P

<150> JP 2002-52310
<151> 2002-02-27

<160> 20

<170> PatentIn version 3.1

<210> 1
<211> 678
<212> DNA
<213> Homo sapiens

<220>
<221> CDS
<222> (1)..(678)
<223>

<400> 1

atg gtc acc cac agc aag ttt ccc gcc gcc ggg atg agc cgc ccc ctg 48
Met Val Thr His Ser Lys Phe Pro Ala Ala Gly Met Ser Arg Pro Leu
1 5 10 15

gac acc agc ctg cgc ctc aag acc ttc agc tcc aag agc gag tac cag 96
Asp Thr Ser Leu Arg Leu Lys Thr Phe Ser Ser Lys Ser Glu Tyr Gln
20 25 30

ctg gtg gtg aac gca gtg cgc aag ctg cag gag agc ggc ttc tac tgg 144
Leu Val Val Asn Ala Val Arg Lys Leu Gln Glu Ser Gly Phe Tyr Trp
35 40 45

agc gca gtg acc ggc ggc gag gcg aac ctg ctg ctc agt gcc gag ccc 192
Ser Ala Val Thr Gly Gly Glu Ala Asn Leu Leu Leu Ser Ala Glu Pro
50 55 60

gcc ggc acc ttt ctg atc cgc gac agc tcg gac cag cgc cac ttc ttc 240
Ala Gly Thr Phe Leu Ile Arg Asp Ser Ser Asp Gln Arg His Phe Phe
65 70 75 80

acg ctc agc gtc aag acc cag tct ggg acc aag aac ctg cgc atc cag 288
Thr Leu Ser Val Lys Thr Gln Ser Gly Thr Lys Asn Leu Arg Ile Gln
85 90 95

tgt gag ggg ggc agc ttc tct ctg cag agc gat ccc cgg agc acg cag 336
Cys Glu Gly Gly Ser Phe Ser Leu Gln Ser Asp Pro Arg Ser Thr Gln

100 105 110

ccc gtg ccc cgc ttc gac tgc gtg ctc aag ctg gtg cac cac tac atg 384
Pro Val Pro Arg Phe Asp Cys Val Leu Lys Leu Val His His Tyr Met
115 120 125

ccg ccc cct gga gcc ccc tcc ttc ccc tcg cca cct act gaa ccc tcc 432
Pro Pro Pro Gly Ala Pro Ser Phe Pro Ser Pro Pro Thr Glu Pro Ser
130 135 140

tcc gag gtg ccc gag cag ccg tct gcc cag cca ctc cct ggg agt ccc 480
Ser Glu Val Pro Glu Gln Pro Ser Ala Gln Pro Leu Pro Gly Ser Pro
145 150 155 160

ccc aga aga gcc tat tac atc tac tcc ggg ggc gag aag atc ccc ctg 528
Pro Arg Arg Ala Tyr Tyr Ile Tyr Ser Gly Gly Glu Lys Ile Pro Leu
165 170 175

gtg ttg agc cgg ccc ctc tcc tcc aac gtg gcc act ctt cag cat ctc 576
Val Leu Ser Arg Pro Leu Ser Ser Asn Val Ala Thr Leu Gln His Leu
180 185 190

tgt cgg aag acc gtc aac ggc cac ctg gac tcc tat gag aaa gtc acc 624
Cys Arg Lys Thr Val Asn Gly His Leu Asp Ser Tyr Glu Lys Val Thr
195 200 205

cag ctg ccg ggg ccc att cgg gag ttc ctg gac cag tac gat gcc ccg 672

Gln Leu Pro Gly Pro Ile Arg Glu Phe Leu Asp Gln Tyr Asp Ala Pro

210 215 220

ctt taa 678

Leu

225

<210> 2

<211> 225

<212> PRT

<213> Homo sapiens

<400> 2

Met Val Thr His Ser Lys Phe Pro Ala Ala Gly Met Ser Arg Pro Leu

1 5 10 15

Asp Thr Ser Leu Arg Leu Lys Thr Phe Ser Ser Lys Ser Glu Tyr Gln

20 25 30

Leu Val Val Asn Ala Val Arg Lys Leu Gln Glu Ser Gly Phe Tyr Trp

35 40 45

Ser Ala Val Thr Gly Gly Glu Ala Asn Leu Leu Leu Ser Ala Glu Pro

50 55 60

Ala Gly Thr Phe Leu Ile Arg Asp Ser Ser Asp Gln Arg His Phe Phe
65 70 75 80

Thr Leu Ser Val Lys Thr Gln Ser Gly Thr Lys Asn Leu Arg Ile Gln
85 90 95

Cys Glu Gly Gly Ser Phe Ser Leu Gln Ser Asp Pro Arg Ser Thr Gln
100 105 110

Pro Val Pro Arg Phe Asp Cys Val Leu Lys Leu Val His His Tyr Met
115 120 125

Pro Pro Pro Gly Ala Pro Ser Phe Pro Ser Pro Pro Thr Glu Pro Ser
130 135 140

Ser Glu Val Pro Glu Gln Pro Ser Ala Gln Pro Leu Pro Gly Ser Pro
145 150 155 160

Pro Arg Arg Ala Tyr Tyr Ile Tyr Ser Gly Gly Glu Lys Ile Pro Leu
165 170 175

Val Leu Ser Arg Pro Leu Ser Ser Asn Val Ala Thr Leu Gln His Leu
180 185 190

Cys Arg Lys Thr Val Asn Gly His Leu Asp Ser Tyr Glu Lys Val Thr

195 200 205

Gln Leu Pro Gly Pro Ile Arg Glu Phe Leu Asp Gln Tyr Asp Ala Pro

210 215 220

Leu

225

<210> 3

<211> 21

<212> DNA

<213> Artificial

<220>

<223> an artificially synthesized primer sequence

<400> 3

acctgcagaa gatgccagaa g 21

<210> 4

<211> 21

<212> DNA

<213> Artificial

<220>

<223> an artificially synthesized primer sequence

<400> 4

tcggcatact caatgcgtac a 21

<210> 5

<211> 28

<212> DNA

<213> Artificial

<220>

<223> an artificially synthesized TaqMan probe sequence

<220>

<221> misc_binding

<222> (1)..(1)

<223> Label FAM (6-carboxy-fluorescein)

<220>

<221> misc_binding

<222> (28)..(28)

<223> Label TAMRA (6-carboxy-N,N,N',N'-tetramethylrhodamine)

<400> 5

tgttcacgct gtcagtgaaa accactcg 28

<210> 6

<211> 22

<212> DNA

<213> Artificial

<220>

<223> an artificially synthesized primer sequence

<400> 6

agaccccttc tcacctcttg ag 22

<210> 7

<211> 22

<212> DNA

<213> Artificial

<220>

<223> an artificially synthesized primer sequence

<400> 7

agaggtagga ggtgcgagtt ca 22

<210> 8

<211> 28

<212> DNA

<213> Artificial

<220>

<223> an artificially synthesized TaqMan probe sequence

<220>

<221> misc_binding

<222> (1)..(1)

<223> Label FAM (6-carboxy-fluorescein)

<220>

<221> misc_binding

<222> (28)..(28)

<223> Label TAMRA (6-carboxy-N,N,N',N'-tetramethylrhodamine)

<400> 8

tccccctggt tgttgtagca gcttaact 28

<210> 9

<211> 22

<212> DNA

<213> Artificial

<220>

<223> an artificially synthesized primer sequence

<400> 9

cttcagcatc tctgtcggaa ga 22

<210> 10

<211> 22

<212> DNA

<213> Artificial

<220>

<223> an artificially synthesized primer sequence

<400> 10

atcgtactgg tccaggaact cc 22

<210> 11

<211> 28

<212> DNA

<213> Artificial

<220>

<223> an artificially synthesized TaqMan probe sequence

<220>

<221> misc_binding

<222> (1)..(1)

<223> Label FAM (6-carboxy-fluorescein)

<220>

<221> misc_binding

<222> (28)..(28)

<223> Label TAMRA (6-carboxy-N,N,N',N'-tetramethylrhodamine)

<400> 11

aacggccacc tggactccta tgagaaag 28

<210> 12

<211> 21

<212> DNA

<213> Artificial

<220>

<223> an artificially synthesized primer sequence

<400> 12

ggcagaagca gcgtcagata t 21

<210> 13

<211> 21

<212> DNA

<213> Artificial

<220>

<223> an artificially synthesized primer sequence

<400> 13

tgtgtgtgga ctttccaagc t 21

<210> 14

<211> 28

<212> DNA

<213> Artificial

<220>

<223> an artificially synthesized TaqMan probe sequence

<220>

<221> misc_binding

<222> (1)..(1)

<223> Label FAM (6-carboxy-fluorescein)

<220>

<221> misc_binding

<222> (28)..(28)

<223> Label TAMRA (6-carboxy-N,N,N',N'-tetramethylrhodamine)

<400> 14

tggagacagc catacccatg ttagcaga 28

<210> 15

<211> 21

<212> DNA

<213> Artificial

<220>

<223> an artificially synthesized primer sequence

<400> 15

ctttcttatc cgcgacagct c 21

<210> 16

<211> 21

<212> DNA

<213> Artificial

<220>

<223> an artificially synthesized primer sequence

<400> 16

cactggatgc gtaggttctt g 21

<210> 17

<211> 21

<212> DNA

<213> Artificial

<220>

<223> an artificially synthesized probe sequence

<220>

<221> misc_binding

<222> (1)..(1)

<223> Label FAM (6-carboxy-fluorescein)

<220>

<221> misc_binding

<222> (21)..(21)

<223> Label TAMRA (6-carboxy-tetramethylrhodamine)

<400> 17

cactggatgc gtaggttctt g 21

<210> 18

<211> 21

<212> DNA

<213> Artificial

<220>

<223> an artificially synthesized primer sequence

<400> 18

actattggca acgagcggtt c 21

<210> 19

<211> 22

<212> DNA

<213> Artificial

<220>

<223> an artificially synthesized primer sequence

<400> 19

ggatgccaca ggattccata cc 22

<210> 20

<211> 28

<212> DNA

<213> Artificial

<220>

<223> an artificially synthesized probe sequence

<220>

<221> misc_binding

<222> (1)..(1)

<223> Label FAM (6-carboxy-fluorescein)

<220>

<221> misc_binding

<222> (28)..(28)

<223> Label TAMRA (6-carboxy-N,N,N',N'-tetramethylrhodamine)

<400> 20

cctgaggctc ttttccagcc ttccttct 28

## Description

### Technical Field

The present invention relates to methods of testing for allergic diseases.

### Background Art

Allergic diseases are considered multifactorial diseases. For example, bronchial asthma and atopic dermatitis are caused by the interaction of expression of many different genes, each of which is influenced by various environmental factors. Thus, it has been extremely difficult to identify a specific gene which causes an allergic disease.

The expression of mutated or defective genes, or overexpression or reduced expression of specific genes is thought to be involved in allergic diseases. To elucidate the role of gene expression in diseases, it is necessary to understand how a gene is involved in disease onset and how expression of the gene is altered by external stimulants such as drugs.

Recently, history taking and confirmation of the patient's family history as well as own anamnesis are important in general for diagnosis of allergic diseases. In addition, allergy diagnosis based on more objective information is performed by a method for testing patient's blood samples and a method for observing patient's immune response to allergen. Examples of the former method are the allergen-specific IgE measurement, the leukocyte histamine release test, and the lymphocyte stimulating test. The presence of an allergen-specific IgE is a proof of occurrence of the allergic reaction to the allergen. However, in some patients allergen-specific IgE may not necessarily be detected. Furthermore, the IgE assay principle requires performing tests for all of the allergens necessary for diagnosis. The leukocyte histamine release test and the lymphocyte stimulating test are the methods for observing the immune system reaction toward a specific allergen in vitro. These methods are complicated in operation.

Another known method (latter method) is allergy diagnosis based on the immune response observed when a patient is actually contacted with an allergen. Such a test includes the prick test, the scratch test, the patch test, the intradermal reaction, and the induction test. These tests allow the direct diagnosis of a patient's allergic reaction; however, they are considered highly invasive tests since the patients are actually exposed to allergens.

In addition, test methods for proving the occurrence of an allergic reaction caused by any allergen are also performed. For example, a high serum IgE titer may indicate the occurrence of allergic reaction in the patient. The serum IgE titer corresponds to the total amount of allergen-specific IgE. Though it is easy to determine the total amount of IgE when any allergen is tested, the IgE titer may be reduced in some patients with a disease such as a non-atopic bronchitis.

Therefore, a marker for an allergic disease that is not only less invasive to patients but also capable of readily providing information necessary for diagnosis would be useful. Since such markers are thought to be profoundly involved in disease onset, they may be an important target in the control of allergic symptoms as well as in diagnosis.

### Disclosure of the Invention

Specifically, an objective of the present invention is to provide a marker gene which allows testing for an allergic disease. Another objective of the invention is to provide a method of testing for an allergic disease and a method of screening for a therapeutic agent for an allergic disease, both using the marker gene.

The present inventors extensively studied to achieve the above objectives. They considered that it is possible to find new targets in the treatment of allergic diseases if genes whose expression levels were different between allergic disease patients and healthy subjects were isolated and the involvements of the genes in allergic reactions were clarified.

Based on this idea, the present inventors sought genes whose expression levels were different between atopic dermatitis patients and healthy subjects. Furthermore, peripheral blood mononuclear cells were selected as biological samples to compare the gene expression levels. Stimulations with antigens in atopic disease patients are known to result in secretion of IL-5, IL-6, and IL-10 from peripheral blood mononuclear cells. The peripheral blood mononuclear cells are closely associated with allergic reactions (Jenmalm M. C. et al., Pediatr. Allergy Immunol. 10: 168-177, 1999). In other words, genes whose expression levels are different in peripheral blood mononuclear cells should be closely associated with allergic reactions. Furthermore, since peripheral blood mononuclear cells can be easily obtained, false negative results are expectedly reduced by yielding them in large amounts. This enables detection of low-level gene expression.

The present inventors thought it possible to isolate an allergic reaction-associated gene by observing alterations in the gene expression in such blood cells involved in an immune response system. Based on this concept, the present applicant succeeded in isolating the following genes whose expression levels are altered in blood cells of peripheral blood from patients with pollinosis, and filed the following patent applications:

Pollinosis-associated gene 373 (WO 00/65046);
Pollinosis-associated gene 419 (WO 00/65045);
Pollinosis-associated gene 513 (WO 00/65049);
Pollinosis-associated gene 581 (WO 00/65048);
Pollinosis-associated gene 795 (WO 00/65050);
Pollinosis-associated gene 627 (WO 00/65051);
Pollinosis-associated gene 441 (WO 00/73435);
Pollinosis-associated gene 465 (WO 00/73439); and
Pollinosis-asscciated gene 787 (WO 00/73440).

Alternatively, the present applicant isolated B1153, a gene having a different expression level in peripheral blood T cells of patients with an allergic disease as compared with that of healthy subjects, and filed a patent application (WO 02/50269). The B1153 gene has been isolated by a differential display method using samples from healthy subjects and allergic disease patients.
Furthermore, the present inventors explored genes whose expression levels are different in peripheral blood mononuclear cells (hereinafter abbreviated as PBMCs), particularly in T cells, from atopic dermatitis patients.
T cells determine immune response. Thus, it is expected that genes whose expression levels are high in T cells from allergic diseases patients may play important roles in immune response.
Immune response and inflammatory response are regulated by various cytokines including interleukins and interferons. Cytokines function to activate their receptors, and also the downstream "Janus tyrosine kinase (JAK)" and "signal transducer and activator of transcription (STAT)". "Suppressor of cytokine signaling (SOCS)" is an essential factor that negatively regulates the cytokine signal pathway.
To examine the role of molecules of the SOCS family in atopic dermatitis, the present inventors measured the levels of mRNA expression in peripheral blood T cells, and revealed that the expression level of an SOCS3 gene was significantly higher in T cells of atopic dermatitis patients than those of healthy subjects. Based on these findings, the present inventors found that the SOCS3 gene can be used as a marker to test for allergic diseases or to screen for therapeutic agents for allergic diseases, and thus completed the present invention. Specifically, the present invention relates to the following methods of testing for allergy, therapeutic agents for allergic diseases, methods of screening for such agents, allergic disease animal models, and kits to carry out these methods.

[1] A method of testing for an allergic disease, which comprises the steps of:

(a) measuring the expression level of a marker gene in a biological sample from a test subject;
(b) comparing the expression level with that of the marker gene in a biological sample from a healthy subject; and
(c) judging the test subject as being affected with an allergic disease when the expression level of the marker gene in a biological sample from the test subject is found to be significantly elevated,

wherein the marker gene is an SOCS3 gene.
[2] The testing method according to [1], wherein the allergic disease is atopic dermatitis.
[3] The testing method according to [1], wherein the gene expression level is measured by using cDNA PCR.
[4] The testing method according to [1], wherein the gene expression level is measured by detecting a protein encoded by the gene.
[5] The testing method according to [1], wherein the biological sample comprises peripheral blood T cells.
[6] A reagent for testing for an allergic disease, which comprises an oligonucleotide comprising at least 15 nucleotides complementary to a polynucleotide which comprises a nucleotide sequence of an SOCS3 gene or the complementary strand thereof.
[7] A reagent for testing for an allergic disease, which comprises an antibody which recognizes a peptide comprising an amino acid sequence encoded by an SOCS3 gene.
[8] A method for screening for a therapeutic agent for an allergic disease, which comprises the steps of:

(1) contacting a candidate compound with a cell expressing a marker gene;
(2) measuring the expression level of the marker gene; and
(3) selecting a compound which decreases the expression level of the marker gene compared to a control in which the candidate compound has not been contacted,

wherein the marker gene is an SOCS3 gene or a gene functionally equivalent thereto.
[9] The method according to [8], wherein the cell is T cell.
[10] A method of screening for a therapeutic agent for an allergic disease, which comprises the steps of:

(1) administering a candidate compound to a test animal;
(2) measuring the expression level of a marker gene in a biological sample from the test animal; and
(3) selecting a compound which decreases the expression level of the marker gene compared to a control in which the candidate compound has not been administered,

wherein the marker gene is an SOCS3 gene or a gene functionally equivalent thereto.
[11] A method of screening for a therapeutic agent for an allergic disease, which comprises the steps of:

(1) contacting a candidate compound with a cell carrying a vector comprising a transcriptional control region of a marker gene and a reporter gene that is expressed under the control of the transcriptional control region;
(2) measuring the activity of the reporter gene; and
(3) selecting a compound which decreases the expression level of the reporter gene compared to a control in which the candidate compound has not been contacted,

wherein the marker gene is an SOCS3 gene or a gene functionally equivalent thereto.
[12] A method of screening for a therapeutic agent for an allergic disease, which comprises the steps of:

(1) contacting a candidate compound with a protein encoded by a marker gene;
(2) measuring the activity of the protein; and
(3) selecting a compound which decreases the activity of the protein compared to a control in which the candidate compound has not been contacted,

wherein the marker gene is an SOCS3 gene or a gene functionally equivalent thereto.
[13] A therapeutic agent for an allergic disease, which comprises as an active ingredient a compound obtainable by the screening method according to any one of [8], [10], [11], and [12].
[14] A therapeutic agent for an allergic disease, which comprises as a main ingredient an antisense DNA that comprises a sequence complementary to a sequence comprising at least 15 continuous nucleotides of the sense strand sequence of a marker gene, wherein the marker gene is an SOCS3 gene or a gene functionally equivalent thereto.
[15] A therapeutic agent for an allergic disease, which comprises as a main ingredient an antibody which binds to a protein encoded by a marker gene, wherein the marker gene is an SOCS3 gene or a gene functionally equivalent thereto.
[16] Use of a transgenic nonhuman vertebrate, in which the expression level of a marker gene has been increased in T cells, as an allergic disease animal model, wherein the marker gene is an SOCS3 gene or a gene functionally equivalent thereto.
[17] A kit for screening for a therapeutic agent for an allergic disease, the'kit comprising an oligonucleotide comprising at least 15 nucleotides complementary to a polynucleotide comprising the nucleotide sequence of a marker gene or the complementary strand thereof and cells expressing the marker gene, wherein the marker gene is an SOCS3 gene or a gene functionally equivalent thereto.
[18] A kit for screening for a therapeutic agent for an allergic disease, the kit comprising an antibody which recognizes a peptide comprising an amino acid sequence encoded by a marker gene and cells expressing the marker gene, wherein the marker gene is an SOCS3 gene or a gene functionally equivalent thereto.

The present invention also relates to a method for treating an allergic disease which comprises the step of administering a compound of any one of the following (A) to (C). In addition, the present invention relates to the use of a compound of any one of the following (A) to (C) for producing a therapeutic agent for an allergic disease:

(A) a compound obtainable by the screening method according to any one of [8], [10], [11], and [12] above;
(B) an antisense DNA comprising a sequence complementary to a sequence comprising at least 15 continuous nucleotides of the sense strand sequence of an SOCS3 gene; and
(C) an antibody which binds to a protein encoded by an SOCS3 gene.

Furthermore, the present invention relates to a method for producing an allergic disease animal model, which comprises the step of increasing the expression level of an SOCS3 gene or a gene functionally equivalent thereto in T cells in a nonhuman vertebrate. In addition, the present invention relates to the use of a transgenic nonhuman vertebrate, in which the expression level of an SOCS3 gene or a gene functionally equivalent thereto has been increased, as an allergic disease animal model.

The structure of SOCS3 gene, which is to be used as a marker gene in the present invention, is known. A marker gene, SOCS3, used in the present invention is reportedly induced by an inflammatory cytokine such as IL-6 or IFN-$\gamma$, or an anti-inflammatory cytokine such as IL-10, and to downregulate the action of these cytokines (Yasukawa H., Sasaki A. and Yoshimura A. 2000. Negative regulation of cytokine signaling pathway. Annu. Rev. Immunol. 18: 143-164).

T cells that have been differentiated into Th2 cells producing IL-4 and IL-5 are important in allergic reactions. Kubo, who is one of the inventors, and his co-researchers found that Th2 cells specifically expressed SOCS3. Furthermore, SOCS3 transgenic mice were created and observed for functional differentiation of T cells. Differentiation of T cells into Th1 cells was markedly suppressed, while Th2 differentiation was enhanced. A presumptive mechanism is that SOCS3 induced by IL-4 suppresses IL-12 receptor-mediated signaling that enhances Th1 differentiation.

Indeed, differentiation of naive T cells into Th1 cells or Th2 cells is accompanied by predominant expression of a particular SOCS molecule. Increased expression is found for SOCS1 in Th1 cells, and for SOCS3 in Th2 cells. It is thought that IL-4/STAT6 signaling is suppressed in Th1 cells, and IL-12/STAT4 signaling is suppressed in Th2 cells (Egwuagu CE, Yu CR, Zhang M, Mahdi RM, Kim SJ, Gery I., J Immunol 2002 Apr 1; 168(7): 3181-7, Suppressors of cytokine signaling proteins are differentially expressed in Th1 and Th2 cells: implications for Th cell lineage commitment and maintenance).

Based on the above findings, it was considered that one of the causes for allergy onset is Th2 differentiation enhanced by an increase in the expression level of SOCS3 in human peripheral blood T cells.

There are some patent publications relating to SOCS3 which disclose only typical function of SOCS3, i.e. suppression of cytokine signal; there is no data indicating the role of SOCS3 in allergic diseases. Representative examples of these publications are as follows.

·WO200129178-A2, EP0877030A2 (SMITHKLINE BECKMAN CORPORATION)

EPO primary response gene 1 (EPRG1)

These publications describe a gene isolated from a human culture cell line that requires erythropoietin for its proliferation, and the relationship between the gene and many diseases associated with blood diseases. However, there is no data supporting such relationship between the gene and any of the diseases.

·WO09923220-A1 (INCYTE PHERM INC.)

Human suppressor of cytokine signaling (HSCS-1)

This publication describes nucleotide sequence information of the gene, and the relationship between the gene and a wide variety of diseases. However, there is no data supporting such relationship between the gene and particular diseases.

·WO9820023-A1 (HALL INST MEDICAL RES WALTER & ELIZA)

Suppressor of cytokine signaling protein (SOCS)

This publication describes cancers, injuries, immune diseases, and hypertension. However, there is no data supporting the relationship between the gene and such diseases.

Furthermore, expressions of mRNAs of CIS-1, SOCS1, SOCS3, and SOCS5 were measured in peripheral blood T cells of patients having atopic dermatitis. As a result, expression of SOCS3 was significantly high in a patient group, and the high correlation of an expressed amount of SOCS3 with a blood IgE value was also recognized (Naoko NAGATA, Tadahiro OSHIDA, Yoichi SEKI, Yuji SUGITA, Hirohisa SAITO, Masato KUBO, Proceedings of the Japanese Society for Immunology Vol. 32, December 2002, ISSN0919-1984 page 198, "High Expression of Suppressors of Cytokine Signaling-3 (SOCS3) Molecule in Patients Having Allergic Diseases"). This finding was published by the present inventors after filing of the patent application.

Herein, "allergic disease" is a general term for diseases involving allergic reactions. More specifically, this term is defined as a disease for which an allergen is identified, a strong correlation between exposure to the allergen and the onset of the pathological change is demonstrated, and the pathological change has been proven to have an immunological mechanism. Herein, an immunological mechanism means that leukocytes show an immune response to allergen stimulation. Examples of allergens are mite antigens, pollen antigens, etc.

Representative allergic diseases include bronchial asthma, allergic rhinitis, atopic dermatitis, pollinosis, insect allergy, etc. Allergic diathesis is a genetic factor that is inherited from allergic parents to their children. Familial allergic diseases are also called atopic diseases and their inherited causative factor is atopic diathesis. Among atopic diseases, atopic dermatitis is a general term for diseases accompanied with dermatitis symptoms.

As used herein, the term '"SOCS3 gene" refers to human SOCS3 gene identified in Examples and a gene located in the same locus or a gene located in a homologous locus in another organism. For example, in the present invention, an SOCS3 gene includes an arbitrary allele in the same locus as or homologous locus to that of the human SOCS3 gene identified in Examples. Specifically, in the present invention, an SOCS3 gene includes the human SOCS3 gene identified in Examples, its polymorphic variants (including SNPs), mutants, splicing variants, and homologues from other organisms. Since the expressional control for these genes are presumed to be substantially identical or similar to that of the human SOCS3 gene, testings for allergic diseases according to the present invention can be carried out by detecting their expression.

Specifically, in the present invention, an SOCS3 gene is substantially identical to an endogenous gene that comprises the following nucleic acid. An endogenous gene refers to an artificially unmodified gene contained in an organism in nature, and a gene that comprises the same nucleotide sequence is substantially identical to the endogenous gene. Furthermore, herein, "gene" refers to a transcription product or nucleic acid (e.g., DNA, RNA, etc.) encoding the same.

(a) A nucleic acid that comprises a nucleotide sequence selected from the nucleotide sequence of an SOCS3 gene.
(b) A nucleic acid comprising a nucleotide sequence of an SOCS3 gene coding sequence, which contains one or more nucleotide substitutions, deletions, and/or insertions.
(c) A nucleic acid encoding at least 15 continuous amino acids of the amino acid sequence of an SOCS3 protein.
(d) A nucleic acid which encodes a protein comprising the amino acid sequence of an SOCS3 protein containing one or more amino acid substitutions, deletions, and/or insertions.
(e) A nucleic acid which hybridizes under a stringent condition to a nucleic acid containing at least 50 continuous nucleotides of an SOCS3 gene but no nucleotide sequence derived from any sequence other than the SOCS3 gene.

The nucleic acid according to (a) includes those comprising preferably at least 40, more preferably 60, 100, 200, 500, and 1,000 or more continuous nucleotides of the nucleotide sequence of an SOCS3 gene (more preferably its coding region), and most preferably its whole length. Such nucleic acids may include polymorphic variants, mutants, and splicing variants of the gene primarily within the same species. Examples of the nucleic acid according to (a) are those containing the nucleotide sequences of at least 30, more preferably 35, 40, 45, and 50 or more continuous nucleotides of the nucleotide sequence of an SOCS3 gene. Nucleotide sequence is preferably selected from the coding region of an SOCS3 gene, and any desirable numbers of nucleotide sequences may be selected. In the case of selecting a plurality of nucleotide sequences, it is preferable to select nucleic acids comprising the continuous nucleotide sequences at two or more, preferably 3, 4, and 5 or more different sites so that the nucleic acids do not overlap.

The nucleic acid according to (b) includes those comprising the nucleotide sequence of the coding region of an SOCS3 gene' in which preferably 15% or less, more preferably 10%, 8%, 5%, and 1% or less of the whole number of its nucleotides are substituted, deleted, and/or inserted. Such nucleic acids include counterpart genes of other close relative species, polymorphic variants, mutants, and splicing variants thereof. Such nucleic acids are those containing nucleotide sequences having the sequence identity of preferably 85% or more, more preferably 90%, 92%, 95%, and 99% or more to the coding region set forth in SEQ ID NO: 1.

Nucleic acid or amino acid sequence identity can be determined using, for example, a BLAST program (Altschul, S. F. et al., 1990, J. Mol. Biol. 215: 403-410). More specifically, nucleotide sequence identity is determined using the blastn program, while amino acid sequence identity using blastp program, and sequence identity search is conducted, for example, using default parameters with all filters containing Low complexity off at the BLAST website of National Center for Biotechnology Information (NCBI) (Altschul, S. F. et al. (1993) Nature Genet. 3: 266-272; Madden, T. L. et al. (1996) Meth. Enzymol. 266: 131-141; Altschul, S. F. et al. (1997) Nucleic Acids Res. 25: 3389-3402; Zhang, J. & Madden, T. L. (1997) Genome Res. 7: 649-656). For example, using the blast2sequences program (Tatiana, A. et al. (1999) FEMS Microbiol Lett. 174: 247-250) for comparing two sequences, the two sequences can be aligned to determine the sequence identity. In this case, gaps are similarly treated as mismatches to calculate the identity value for the entire coding region of an SOCS3 gene.

The nucleic acid according to (c) includes those encoding preferably at least 20, more preferably, 30, 50, 100, 300, and 500 or more continuous amino acids of the amino acid sequence of an SOCS3 protein, and most preferably its whole length. Such nucleic acids include, similarly as in (a) above, polymorphic variants, mutants, and splicing variants of genes primarily within the same species of organism. Furthermore, the nucleic acid according to (c) includes those comprising partial nucleotide sequences encoding the amino acid sequence of at least 8, more preferably 10, 15, 20, and 25 or more continuous amino acids of an SOCS3 protein at two or more, preferably 3 or more, more preferably 4 and 5 or more sites so that each nucleic acids do not overlap.

The nucleic acid according to (d) includes those encoding the amino acid sequence of an SOCS3 protein in which preferably 15% or less, more preferably 10%, 8%, 5%, and 1% or less of the whole number of its amino acid residues are substituted, deleted, and/or inserted. These nucleic acids may contain untranslated sequences. Such nucleic acids include a counterpart gene of other close relative species, polymorphic variants, mutants, and splicing variants. Such nucleic acids are those encoding proteins comprising amino acid sequences having the sequence identity of preferably 85% or more, more preferably 90%, 92%, 95%, and 99% or more to an SOCS3 protein. Treating gaps similarly as mismatches, the identity value relative to the whole amino acid sequence of an SOCS3 protein is calculated. Amino acid sequence identity can be determined according to the above-described method.

The nucleic acid'according to (e) includes those hybridizing to nucleic acids comprising preferably at least 80, more preferably 100, 120, and 200 or more continuous nucleotides of an SOCS3 gene (more preferably its coding region), and substantially comprising no other nucleotide sequence than that of the SOCS3 gene (more preferably its coding region) under the stringent condition. Such nucleic acids may be those hybridizing to probes that have been prepared, for example, using the nucleic acid comprising the nucleotide sequence of an SOCS3 gene as a template, under the stringent condition. Probes consisting of 50 to several hundreds of nucleotides can be synthesized, for example, by the DNA synthesis method, or by the random prime method, the nick translation method, and the PCR method.

The stringent condition for the nucleic acid according to (e) is that hybridization is carried out in a hybridization solution preferably comprising NaCl in the range of about 0.5 to about 0.9 M at 60°C, preferably at 62°C, more preferably at 65°C, and then washing is performed at the same temperature as for hybridization in 1 x SSC, preferably in 0.5 x SSC, more preferably in 0.2 x SSC, still more preferably in 0.1 x SSC for 1 h. In this case, the temperature conditions of hybridization and washing that greatly influence the stringency can be adjusted according to the melting temperature (Tm) of the probe, which varies depending on the ratio of constituent nucleotides of the probe to the base pairs to which the probe hybridizes, the length of the probe, and the composition (concentrations of salts and formamide) of a hybridization solution. Considering these conditions, those skilled in the art can empirically set up the appropriate condition to confer the equivalent stringency. As a hybridization solution, for example, 4 x SSC, preferably ExpressHyb™ Hybridization Solution (Clontech) or such, may be used.

A method of testing for an allergic disease of the present invention comprises the steps of measuring the expression level of an SOCS3 gene in a biological sample from a test subject, and comparing the measured value to that of the normal healthy subject. Because of comparing both values, when the expression level is enhanced compared to that of the normal healthy subject, the test subject is judged to be affected with an allergic disease. For the comparison of expression levels, the standard value is usually set up based on the expression level of the marker gene in the normal healthy subject. Based on this standard value, a permissible range is set, for example, to be the standard value ± 2 S.D. The method of setting up the standard value and permissible range based on measured values of the marker gene is well known in the art. When the expression level of the marker gene in a test subject is higher than the permissible range, that subject is judged to be affected with an allergic disease, while when the expression level is within the permissible range, that subject is less likely to be affected with the allergic disease. Herein, "marker gene" means an SOCS3 gene that can serve as a marker of allergic diseases.

The marker gene is not limited to an SOCS3 gene alone; it can be combined with other marker genes for allergic diseases. The testing accuracy can be increased by measuring a combination of plural genes as the marker gene. Since patients with an allergic disease such as atopic dermatitis form a heterogeneous population, a more accurate diagnosis can be performed using a plurality of genes as the marker.

In this invention, expression levels of marker genes include levels of transcription of these genes to mRNA and translation into proteins. Therefore, a method of testing for an allergic disease of this invention is performed comparing the mRNA expression level of the marker genes, or the expression level of proteins encoded by the marker genes.

In testings of allergic diseases of this invention, the expression levels of marker genes can be measured according to known genetic analysis methods. Specifically, one can use a hybridization technique using nucleic acids that hybridize to these genes as probes, or a gene amplification technique using DNAs that hybridize to genes used in this invention as primers.

The probes or primers used for testings of this invention can be designed based on the nucleotide sequences of the marker genes. For example, the nucleotide sequence of human SOCS3 gene is known as GenBank Acc. No. AB006967. The nucleotide sequence of human SOCS3 gene is shown in SEQ ID NO: 1 and the amino acid sequence encoded by this nucleotide sequence is shown in SEQ ID NO: 2.

In general, genes of higher animals are often accompanied by polymorphism. Many molecules produce isoforms comprising different amino acid sequences from each other during the splicing process. Marker genes of the present invention include any genes associated with allergy and having a similar activity to that of the marker genes, even though they carry mutation in the nucleotide sequence due to polymorphism or isoform.

As a primer or probe, a polynucleotide comprising the nucleotide sequence of a marker gene or at least 15 nucleotides that are complementary to the complementary strand of the polynucleotide, can be used. Herein, the term "complementary strand" means one strand corresponding to the other strand of a double stranded DNA composed of A:T (U for RNA) and G:C base pairs. In addition, "complementary" means not only those completely complementary to a region of at least 15 continuous nucleotides, but also having a homology of at least 70%, preferably at least 80%, more preferably 90%, and even more preferably 95% or higher. The homology between nucleotide sequences can be determined by the algorithm, BLAST, etc.

Such polynucleotides are useful as a probe for detecting a marker gene, or as a primer to amplify a marker gene. When used as a primer, those polynucleotides comprise usually 15 bp to 100 bp, preferably 15 bp to 35 bp of nucleotides. When used as a probe, DNAs comprising the whole sequence of a marker gene (or its complementary strand), or its partial sequence that contains at least 15-bp nucleotides. When used as a primer, its 3' region must be complementary to a marker gene, while the 5' region can be linked to a restriction enzyme-recognition sequence or tag.

"Polynucleotides" used in the present invention may be either DNA or RNA. These polynucleotides may be either synthetic or naturally occurring. In addition, DNA used as a probe for hybridization is usually labeled. Examples of labeling methods are those as described below. Herein, the term "oligonucleotide" means a polynucleotide with relatively low degree of polymerization. Oligonucleotides are included in polynucleotides.

· nick translation labeling using DNA polymerase I;
· end labeling using polynucleotide kinase;
· fill-in end labeling using Klenow fragment (Berger, SL., Kimmel, AR. (1987) Guide to Molecular Cloning Techniques, Method in Enzymology, Academic Press; Hames, BD., Higgins, SJ. (1985) Genes Probes: A Practical Approach. IRL Press; Sambrook, J., Fritsch, EF., Maniatis, T. (1989) Molecular Cloning: a Laboratory Manual, 2nd Edn. Cold Spring Harbor Laboratory Press);
· transcription labeling using RNA polymerase (Melton, DA., Krieg, PA., Rebagkiati, MR., Maniatis, T. , Zinn, K. , Green, MR. (1984) Nucleic Acid Res., 12, 7035-7056); and
· non-isotopic labeling of DNA by incorporating modified nucleotides (Kricka, LJ. (1992) Nonisotopic DNA Probing Techniques. Academic Press).

For testing of an allergic disease using hybridization techniques such as Northern hybridization, dot blot hybridization, or the DNA microarray technique may be used. Furthermore, gene amplification techniques, such as the RT-PCR method maybe used. By using the PCR amplification monitoring method during the gene amplification step in RT-PCR, one can achieve more precise quantitative analysis for the expression of a gene of the present invention.

In the PCR gene amplification monitoring method, the detection target (DNA or reverse transcript of RNA) is hybridized to probes that are dual-labeled at both ends with different fluorescent dyes whose fluorescences cancel each other out. When the PCR proceeds and Taq polymerase degrades the probe by its 5'-3' exonuclease activity, the two fluorescent dyes become distant from each other and thus the fluorescence can be detected. The fluorescence is detected in real time. By simultaneously measuring a standard sample in which the copy number of the target is known, it is possible to determine the copy number of the target in a subject sample at the cycle number where PCR amplification is linear (Holland, P. M. et al., 1991, Proc. Natl. Acad. Sci. USA 88: 7276-7280; Livak, K. J. et al., 1995, PCR Methods and Applications 4(6): 357-362; Heid, C. A. et al., 1996, Genome Research 6: 986-994; Gibson, E. M. U. et al., 1996, Genome Research 6: 995-1001). For the PCR amplification monitoring method, for example, ABI PRISM7700 (PE Biosystems) may be used.

A method of testing for an allergic disease in the present invention can also be carried out by detecting a protein encoded by a marker gene. Hereinafter, a protein encoded by a marker gene is referred to as a marker protein. For such test methods the Western blotting method, the immunoprecipitation method, and the ELISA method may be employed using an antibody that binds to a marker protein.

Antibodies that bind to a marker protein used in the detection may be produced by techniques known to those skilled in the art. Antibodies used in the present invention may be polyclonal or monoclonal (Milstein, C. et al., 1983, Nature 305 (5934): 537-40). For example, polyclonal antibodies against a marker protein may be produced by collecting blood from mammals sensitized with the antigen, and separating serum from the blood using known methods. As polyclonal antibodies, serum containing polyclonal antibodies itself may be used. As the occasion demands, a fraction containing polyclonal antibodies can be further isolated from this serum. In addition, monoclonal antibodies may be obtained by isolating immune cells from mammals sensitized with the antigen, fusing these cells with myeloma cells and such, cloning hybridomas thus obtained, and collecting antibodies as monoclonal antibodies from the culture of the hybridomas.

For detecting a marker protein, these antibodies may be appropriately labeled. Instead of labeling the antibodies, a substance that specifically binds to the antibodies, such as protein A or protein G, may be labeled to indirectly detect a marker protein. One example of the detection method is ELISA.

Protein or its partial peptide used as an antigen may be obtained by inserting its gene or portion thereof into an expression vector, introducing the vector into an appropriate host cell to produce a transformant, culturing the transformant to express the recombinant protein, and purifying the expressed recombinant protein from the culture or the culture supernatant. Alternatively, amino acid sequences encoded by these genes, or oligopeptides comprising portions of the amino acid sequence encoded by the full-length cDNA are chemically synthesized to serve as the antigen.

Furthermore, in the present invention, a testing for an allergic disease can be performed by detecting not only the expression level of a marker gene but also the activity of a marker protein in a biological sample as an index. Activity of a marker protein means a biological activity intrinsic to each protein. The activity of a marker protein can be detected by known method.

Normally, in the testing method of this invention, a biological sample from a subject is used as a test sample. A biological sample such as peripheral blood T cells can be used. The method of obtaining T cells from the peripheral blood is well known in the art. That is, PBMC can be obtained by centrifuging diluted peripheral blood using Ficoll. A testing method of the present invention can be carried out by measuring a marker gene or a marker protein in PBMC thus isolated as a sample. PBMC comprises lymphocytes and monocytes. However, as shown in the Examples, among these blood cells the marker gene is highly expressed in T cells. Therefore, the presence of cells other than T cells hardly affects the measurement result of the marker gene or the marker protein. Alternatively, T cells can be isolated by allowing them to be specifically adsorbed by microbeads to which the anti-CD3 antibody has been immobilized.

The intracellular mRNA of a marker gene or a marker protein can be measured in disintegrated blood cells. It is also possible to measure a marker protein in blood. For the preparation of T cell lysate and extraction of mRNA, kits such as an RNeasy Mini® (Qiagen) and ISOGEN® (Nippon Gene) on the market may be conveniently used.

The measured value of the expression level of a marker gene in T cells can be corrected for by known methods. The changes in the gene expression level in cells can be compared using the corrected values. The measured value of the expression level of genes that are to be used as markers in the present invention is corrected based on the measured value of the expression level of the genes (housekeeping genes) that are expressed in T cells and do not largely fluctuate in their expression levels regardless of the condition of the cell. Examples of such genes are β-actin gene and glyceraldehyde 3-phosphate dehydrogenase (GAPDH) gene.

A marker gene used in the present invention is expressed at a high level in T cells from patients with atopic dermatitis compared to that in normal healthy subjects. Therefore, the testing for an allergic disease such as atopic dermatitis can be performed using the expression level of the marker gene as a marker.

A testing for an allergic disease according to the present invention comprises those as described below. Even a patient who cannot be judged as having an allergic disease by the ordinary testing in spite of showing symptoms of atopic dermatitis can be easily judged as an allergic disease patient by a testing of this invention. More specifically, the elevation of the marker gene expression in patients with symptoms suspect of an allergic disease indicates that the symptoms would probably be those of an allergic disease. There are two types of dermatitis symptoms: one type being caused by an allergic reaction while the other type is not. Since the treatment methods for these two types are entirely different, diagnosis of the cause of the dermatitis symptom is a very important step in the treatment. A testing method of this invention can provide extremely important information for identifying the cause of dermatitis.

Alternatively, the testing method enables to judge whether allergic symptoms are being ameliorated or not. The expression level of a marker gene of this invention increases in T cells of patients with an allergic disease. T cells are the cells playing a central role in immune response. Therefore, a gene whose expression level is enhanced in T cells that regulate immune response is useful as a marker for treatment effectiveness. More specifically, the increased expression level of a marker gene in patients diagnosed as having an allergic disease indicates that the allergic symptoms would be in progress.

The present invention also relates to the use of a transgenic nonhuman animal in which the expression level of a marker gene SOCS3 or a gene functionally equivalent thereto is elevated in T cells, as an allergic disease animal model. The allergic disease animal model is useful in clarifying changes *in vivo* in allergy. Furthermore, an allergic disease animal model of this invention is useful in the assessment of therapeutic agents for an allergic disease.

The present invention demonstrated the elevated expression level of a marker gene in T cells. Therefore, animals in which the expression level of an SOCS3 gene or a gene functionally equivalent thereto is artificially elevated in T cells can be utilized as the allergic disease animal model. Herein, transgenic animals refer to animals genetically modified by insertion, substitution, and/or deletion of one or more nucleotides in their genomic DNAs. This allergic disease animal model is useful in clarifying changes *in vivo* in allergy. Furthermore, an allergic disease animal model of this invention is useful in assessing and screening for therapeutic agents for an allergic disease. In this case, the increased expression level in T cells includes the increased expression level of a marker gene in the whole blood cells. That is, the expression level elevation of a marker gene includes that in not only T cells but also blood cells as a whole or the whole body.

Genes functionally equivalent to an SOCS3 gene used in the present invention refer to those encoding proteins having the activity equivalent to that of the protein (marker protein) encoded by the marker gene SOCS3. Such genes include artificial variants of human SOCS3 gene or its counterparts from other organisms. For example, in the case of inserting a gene into an expression vector, deletion of nucleotides corresponding to several amino acid residues at the N- or C-terminus, and addition of nucleotides corresponding to a tag peptide sequence or other amino acid sequence derived from the expression vector are frequently performed. The resulting proteins are different in the amino acid sequence from the endogenous protein in natural cells, but their activities are substantially equivalent to that of the endogenous protein. Herein, genes encoding such proteins are referred to as genes functionally equivalent to the endogenous gene.

One example of the activities of a marker protein used in this invention is inhibition the Janus kinase (JAK). For example, a gene encoding the human SOCS3 protein in which one or more amino acids are deleted, inserted, and/or substituted and which maintains the JAK inhibition activity, can be used to prepare a transgenic animal model of this invention. Such modified proteins should have a homology of, for example, 90% or more, preferably 95% or more, and still more preferably 99% or more to the amino acid sequence of the human SOCS3 protein. Furthermore, genes which comprise nucleotide sequences hybridizing to DNAs containing the nucleotide sequence of the SOCS3 gene under stringent conditions, and encode proteins having the activity to inhibit JAK, can also be used as genes functionally equivalent to the SOCS3 gene.

Alternatively, genes encoding proteins having a homology of, for example, 90% or more, preferably 95% or more, and more preferably 99% or more to the amino acid sequence of the human SOCS3 protein are included in the genes functionally equivalent to the SOCS3 gene. Genes which can be amplified using oligonucleotides comprising the nucleotide sequences set forth in SEQ ID NOs: 1 and 2 used in Examples as the primers, and which shows increased expression in T cells, are also functionally equivalent genes.

A animal model of this invention can be used as a model for an allergic disease in a method comprising the steps of: (a) detecting a phenotype of the animal model; and (b) correlating the difference of the phenotype of said animal model from the corresponding phenotype of a control animal, whose expression level of a marker gene in T cells is lower compared to that of said animal model, with an allergic disease. Examples of the control animals include non-transgenic animal having the same genetic background as that of said animal model and a transgenic animal in which an empty vector has been introduced. The phenotype may be any desired phenotypes including allergic symptoms such as dermatitis, rhinitis, and asthma, or the activation of immunocytes or changes in gene expression.

In the present invention, it is highly significant to assess roles of the marker gene SOCS3 and effects of drugs targeting this gene using transgenic animals in which the expression level of the marker gene is elevated in T cells, as the allergic disease animal model. Furthermore, allergic disease animal models according to the present invention are useful not only in screening for pharmaceutical agents for the treatment or prophylaxis of an allergic disease as described below but also in elucidating the mechanism of allergic diseases, and furthermore, testing the safety of screened compounds. For example, if the allergic disease animal models according to this invention either develop atopic dermatitis or allergic asthma, or show changes in measured values associated with any allergic diseases, it is possible to construct a system for screening for compounds that can cure the allergic conditions.

Herein, the elevation of the expression level is attributed to any of the states that: a target gene introduced as an exogenous gene is allowed to be expressed; transcription of a gene inherent in the host and/or its translation into protein are/is increased; and, decomposition of the protein that is a translation product is suppressed. The gene expression level can be confirmed by, for example, quantitative PCR as described in Examples. Furthermore, the expression level or activity of the translation product protein can be confirmed by comparing it with that in a normal state.

A typical transgenic animal is an animal which has been transfected with a gene of interest and allowed to express it. In another type of transgenic animals the half-life of mRNA may be extended by removing a sequence that renders RNA unstable, from the untranslated region (UTR) of mRNA. Furthermore, in another type of transgenic animals, a mutation is introduced into the coding region of a marker gene to increase its activity or modify the amino acid sequence of the gene product protein to be hardly decomposed. Examples of mutation in the amino acid sequence are substitution, deletion, insertion, or addition of amino acid residues. In addition, mutation in the transcriptional regulatory region of a marker gene also enables to enhance the expression of the gene.

Methods for obtaining transgenic animals targeting a specific gene are well known in the art. That is, a transgenic animal can be obtained by a method where the gene and ovum are mixed and treated with calcium phosphate; a method where the gene is directly introduced into pronuclei of oocyte under a phase contrast microscope using a micropipette (the microinjection method, U.S. Patent No. 4,873,191); a method where the gene is introduced into embryonic stem cells (ES cells) ; etc. Furthermore, other methods include a method where ovum is infected with a gene-inserted retroviral vector and a sperm vector method where a gene is introduced into ovum mediated by sperm. The sperm vector method is a gene recombination technique for introducing an exogenous gene and performed by allowing an exogenous gene to adhere to a sperm or to be incorporated in a sperm by the electroporation method or such and fertilizing ovum with the sperm (M. Lavitranoet, et al., Cell, 57, 717, 1989).

Transgenic animals used as an allergic disease animal model of the present invention can be produced using all the vertebrates except for humans. More specifically, transgenic animals in which various genes have been introduced and their expression levels are modified are produced using vertebrates such as mice, rats, rabbits, miniature pigs, goats, sheep, monkeys, and cattle.
Furthermore, the present invention relates to a method of screening for a therapeutic agent for an allergic disease. In this invention, the expression level of a marker gene significantly increases in T cells of allergic disease patients. Therefore, it is possible to obtain a therapeutic agent for an allergic disease by selecting a compound capable of reducing the expression level of the gene. Compounds that reduce the expression level of the gene inhibit any steps of transcription or translation of the gene or activity expression of a protein.
A method of screening for a therapeutic agent for an allergic disease of this invention can be carried out either in vivo or in vitro. This screening method can be carried out according to the steps as described below. Marker genes in the screening method of this invention includes, in addition to an SOCS3 gene, any genes functionally equivalent thereto.

(1) administering a candidate compound to a test animal;
(2) measuring the expression level of a marker gene in a biological sample from the test animal; and
(3) selecting a compound that reduces the expression level of the marker gene, compared to a control in which the candidate compound has not been administered.

As a test animal in a screening method of the present invention, for example, an allergic disease animal model may be used. Such an allergic animal model is well known in the art. For example, as a model closely resembling human atopic dermatitis, a spontaneous dermatitis model using NC/Nga mouse has been reported. Administration of the mite antigen (5 µg/ear) into the auricle of this mouse 8 times in total at 2 to 3 days intervals enables the induction of symptoms that closely resemble human atopic dermatitis after two weeks. A screening according to this invention can be conducted by administering a candidate compound to this system and monitoring changes in the expression level of a marker gene of this invention.
Thus, effects of the drug candidate compound on the expression level of a marker gene can be detected by administering a drug candidate compound to a test animal, and monitoring the action of the compound toward the expression of the marker gene in a biological sample from the test animal. Changes in the expression level of the marker gene in biological samples from test animals can be monitored by a method similar to above-described testing method of this invention. Furthermore, based on the detection results, drug candidate compounds can be screened by selecting compounds that reduce the expression level of the marker gene.
More specifically, a screening according to the present invention can be carried out by comparing the expression level of a marker gene in a biological sample collected from a test animal to that in a control in which a candidate compound has not been administered. Examples of the biological sample include whole blood, PBMC, and T cells. Methods for collecting and preparing these biological samples are known in the art.
These screening methods enable selection of drugs involved in the marker gene expression in various ways. More specifically, for example, drug candidate compounds having the following actions can be found:

reduce the transcriptional activity of the marker gene;
reduce the translation level from the transcript of the marker gene;
inhibit the activity of translation product of the marker gene; and
reduce the stability of the transcript of the marker gene or accelerate its decomposition.

The above-described screening methods of the present invention also include a method comprising the step of stimulating test animals with an allergen before and/or after the administration of a candidate compound. When the allergen stimulation is performed prior to the administration of a candidate compound, it is possible to detect the action of a candidate compound that inhibits immune response occurring after the allergen stimulation. Compounds obtainable by this screening method are expected to have therapeutic effects on an allergic disease. When allergen stimulation is conducted after the candidate compound administration, it is possible to detect the activity of a candidate compound that suppresses initiation of immune response occurring by the allergen stimulation. Compounds obtainable by this screening method are expected to have prophylactic effects on an allergic disease. An allergen usable in a screening method of this invention includes allergenic substances known in the art. More specifically, allergens well known in the art include mite, house dust, plant pollen, proteins derived from diverse foods. These allergens may be derived from the nature or synthesized by the gene recombination technique and the like method. Furthermore, allergens may be protein fragments. Methods for preparing a purified allergen are also well known in the art.
Examples of *in vitro* screening include a method in which cells expressing a marker gene are contacted with a candidate compound to select a compound that reduces the expression level of the marker. gene. This screening may be carried out according to the steps of:

(1) contacting a candidate compound with cells expressing a marker gene;
(2) measuring the expression level of the marker gene; and
(3) selecting a compound that reduces the expression level of the marker gene, compared to a control in which the candidate compound has not been contacted.

In the present invention, cells expressing a marker gene can be obtained by inserting the marker gene to an appropriate expression vector, and introducing the vector into a suitable host cell. Any vectors and host cells may be used as long as they are able to express the gene of this invention. Examples of host cells in the host-vector system include *Escherichia coli*, yeast, insect cells, and animal cells, and vectors usable for respective host cells can be appropriately selected.

The vectors may be introduced into the host by a biological method, a physical method, a chemical method, etc. Examples of the biological method are a method using virus vectors, a method using a specific receptor, cell-fusion method (HVJ (Sendai virus)), polyethylene glycol (PEG) method, electric cell fusion method, microcell fusion method (chromosome transfer)). Examples of the physical method are microinjection method, electroporation method, and a method using the gene particle gun (gene gun). Examples of the chemical method are calcium phosphate precipitation method, liposome method, DEAE-dextran method, protoplast method, erythrocyte ghost method, erythrocyte membrane ghost method, and microcapsule method.

As cells expressing a marker gene, an established T cell line Molt4 and human acute leukemia T cell line Jurkat (ATCC Number TIB-152), are preferred for a screening method of the present invention. These cells are commercially available from ATCC.

In a screening method of this invention, first a candidate compound is added to the cell strain. Then, the expression level of a marker gene in the cell strain is measured to select a compound that reduces the expression level of the gene. In the screening method of this invention, the expression level of a marker gene can be compared not only based on the expression level of a protein encoded by the gene but also based on the corresponding mRNA detected. For comparing the expression levels using mRNA, an mRNA sample is prepared as described above in place of the preparation of a protein sample. Detection of mRNA and protein can be performed by known methods as described above.

Furthermore, based on the disclosure of this invention, it is possible to obtain the transcriptional regulatory region for a marker gene of this invention and construct a reporter assay system. Reporter assay system means an assay system for screening for a transcriptional regulatory factor that acts on the transcriptional regulatory region using the expression level of a reporter gene located downstream of the transcriptional regulatory region as an index.

That is, this invention relates to a method of screening for a therapeutic agent for an allergic disease, the method comprising the steps of:

(1) contacting a candidate compound with cells transfected with a vector containing a transcription regulatory region of a marker gene and a reporter gene that is expressed under the control of the transcription regulatory region,
(2) measuring the activity of the reporter gene, and
(3) selecting a compound that lowers the reporter gene expression level, compared to a control in which the candidate compound has not been contacted,

wherein the marker gene is a SOCS3 gene or a gene functionally equivalent thereto.

Examples of the transcription regulatory region are promoters, enhancers, and furthermore, CAAT box and TATA box, which are normally seen in the promoter region. Examples of the reporter genes include chloramphenicol acetyltransferase (CAT) gene, luciferase gene, and growth hormone genes.

With respect to the transcriptional regulatory region of the mouse and rat SOCS3 genes, it has been reported that transcription is induced by growth hormone and IL-6, and that STAT-1/STAT-3 binding sequence as well as characteristic cis element exist in the transcriptional regulatory region (Proc Natl Acad Sci USA 1999 96(12): 6964-9, Autoregulation of pituitary corticotroph SOCS-3 expression: characterization of the murine SOCS-3 promoter. Auernhammer CJ, Bousquet C, Melmed S.; Eur J Biochem 2000 Oct; 267(19): 5849-57, Regulation of expression of the rat SOCS-3 gene in hepatocytes by growth hormone, interleukin-6 and glucocorticoids mRNA analysis and promoter characterization. Paul C, Seiliez I, Thissen JP, Le Cam A.; J Biol Chem 2002 Jan 18; 277(3): 2345-52, Regulation of Socs gene expression by the proto-oncoprotein GFI-1B: two routes for STAT5 target gene induction by erythropoietin. Jegalian AG, Wu H.). Similar experiments may also be conducted in humans. That is, an assay system for measuring the expression level of SOCS3 can be constructed by linking a reporter gene such as luciferase and CAT genes downstream of the transcriptional regulatory region of SOCS3.

For example, the transcription regulatory region used in the screening of this invention can be obtained as follows. That is, first, a human genome DNA library such as BAC library and YAC library is screened for the nucleotide sequences of marker genes disclosed in this invention by PCR or hybridization to obtain a genomic DNA clone containing the cDNA sequence. Based on the obtained genomic DNA sequence, the transcription regulatory region of cDNA disclosed in this invention is estimated, and the transcription regulatory region is obtained. A reporter construct is constructed by cloning the obtained transcription regulatory region so that it is positioned upstream of the reporter gene. The obtained reporter construct is used to transfect a cultured cell strain to obtain a transformant for screening. By contacting the candidate compounds with this transformant, compounds that regulate the expression of reporter genes can be screened.

Furthermore, cells containing a reporter gene linked to function under the control of the transcriptional regulatory region of a marker gene may be cells of a so-called knockin animal. A knockin animal refers to a transgenic animal in which an exogenous gene has been inserted into the protein-coding region of the endogenous target gene. Since this knocked-in exogenous gene is inserted downstream of the transcriptional regulatory region of the target gene, it is expressed under a similar expression control to that for the endogenous target gene. It is possible to carry out screening using the knockin animal or cells obtained from it using the reporter gene as a knockin gene. A screening method using a knockin animal comprises the steps of: (a) administering a candidate compound to an animal in which a reporter gene has been knocked-in to the marker gene site, (b) measuring the expression level of the reporter gene, and (c) selecting a compound capable of reducing the expression level of the reporter gene compared to a control in which the candidate compound has not been administered. For example, the expression level of the reporter gene in leukocytes, more preferably in T cells of the knockin animal to which a candidate compound is administered or not administered, are measured to select a compound capable of reducing the expression level. Furthermore, a screening method using cells from the knockin animal comprises the steps of: (a) contacting a candidate compound with cells from an animal in which a reporter gene has been knocked-in to the marker gene site, (b) measuring the expression level of the reporter gene, and (c) selecting a compound capable of reducing the expression level of the reporter gene compared to that in a control in which the candidate compound has not been contacted. For example, the expression level of the reporter gene in leukocytes, more preferably in T cells obtained from a knockin animal are measured in the presence or absence of a candidate compound to select a compound capable of reducing the expression level. These screenings are included in screening methods of the present invention. In the screening using an individual animal in particular, it is preferable to perform the screening using a knockin heterozygote in which one allele is left intact so as to express the marker gene, while the reporter gene has been knocked in the other allele, to avoid the complete deletion of the function intrinsic to the marker gene.

As an SOCS3 expression inhibitor a dominant negative mutant may be used. More specifically, an SOCS3 gene having a point mutation in the N-terminal kinase inhibitory region (KIR) has been well known to function as a dominant negative mutant to inhibit the SOCS3 expression.

An *in vitro* screening method according to the present invention can be performed based on the activity of a marker protein. That is, the present invention relates to a method of screening for therapeutic agents for an allergic disease, the method comprising the steps of:

(1) contacting a candidate compound with a protein encoded by a marker gene,
(2) measuring the activity of said protein, and
(3) selecting a compound capable of reducing the activity of said protein compared to a control in which said candidate compound has not been contacted with said protein,

wherein the marker gene is either SOCS3 or a gene functionally equivalent thereto.

Herein, genes functionally equivalent to the marker gene include an endogenous SOCS3 gene that has been artificially modified as described above.

Such screening can be conducted, for example, by allowing host cells to exogenously express the marker gene and measuring the intracellular activity of the marker protein. In this case, the marker gene may be inserted into an expression vector, and the resulting recombinant vector is introduced into appropriate hosts such as mammalian cells. The vectors can be introduced into the host via biological, physical, or chemical methods. Examples of the biological method are a method using viral vectors, a method using a specific receptor, and cell fusion method (via HVJ (Sendai virus) , polyethylene glycol (PEG) method, electric cell fusion method, and microcell fusion method (chromosome transfer)). Examples of the physical method are a microinjection method, electroporation method, and a method using the gene particle gun (gene gun). Examples of the chemical method are a calcium phosphate precipitation method, liposome method, DEAE-dextran method, protoplast method, red cell ghost method, red cell membrane ghost method, and microcapsule method.

A marker protein SOCS3 used in this invention has been reported to negatively regulate the action of anti-inflammatory cytokine such as IL-10 (Yasukawa, H., Sasaki, A., and Yoshimura, A. 2000. Negative regulation of cytokine signaling pathway. Annu. Rev. Immunol. 18: 143-164). In addition, SOCS3 has a function to induce the differentiation of T cells into Th2.

Furthermore, SOCS3 has the activity to inhibit the signaling from a receptor by binding to the receptor itself or its downstream factor, Janus family of protein tyrosine kinase protein (JAK). Therefore, the inhibition activity of SOCS3 protein can be measured by using the binding of SOCS3 to its receptor or the JAK protein as an indicator. Furthermore, in the JAK and STAT systems, the SOCS3 activity can be measured using the kinase activity of a kinase JAK or the transcription activity of a transcription factor STAT as the indicator.

For example, JAK2 and STAT4 act downstream of the IL-12 receptor that is important for the Th1 differentiation. SOCS3 binds to JAK2 to inhibit its kinase activity (Sasaki, A., Yasukawa, H., Suzuki, A., Kamizono, S., Syoda, T., Kinjyo, I., Sasaki, M., Johnston, J.A., Yoshimura, A., Genes Cells 1999 Jun. 4 (6): 339-51, Cytokine-inducible SH2 protein-3 (CIS3/SOCS3) inhibits Janus tyrosine kinase by binding through the N-terminal kinase inhibitory region as well as SH2 domain) . Therefore, the SOCS3 activity can be assessed by measuring the binding of SOCS3 to JAK2 or the kinase activity of JAK2. Furthermore, inhibition of JAK2 activity results in inhibition of the transcription activity of STAT4. The transcription activity of STAT4 can be easily measured in a reporter assay system using the STAT4-responsive element as a promoter. Thus, using the above-described method, the SOCS3 activity to inhibit the signaling downstream of IL-12 can be measured.

With these activities as the indicators, it is possible to screen for compounds having the activity to inhibit these respective activities. Compounds thus obtained inhibit the SOCS3 activity, leading to the regulation of allergic immune response through the inhibition of the marker protein whose expression in T cells has been induced.

Candidate test compounds used in this screening include compound preparations synthesized by existing chemical methods such as steroid derivatives, compound preparations synthesized by combinatorial chemistry, mixtures of multiple compounds such as extracts from animal or plant tissues or microbial cultures, and their purified preparations, etc.

A polynucleotide, antibody, cell strain, or animal model necessary for various screening methods according to this invention can be previously combined into a kit. More specifically a kit may be composed of a cell expressing a marker gene and a reagent to measure the expression level of the marker gene. The reagent for measuring the expression level of the marker gene includes a polynucleotide comprising the nucleotide sequence of at least one marker gene, or at least 15-nucleotide-long oligonucleotides comprising a nucleotide sequence complementary to the complementary strand of the marker gene sequence. Alternatively, the reagent may be an antibody that recognizes a peptide comprising the amino acid sequence of at least one marker protein. These kits may also be packaged with a substrate compound used for the detection of the marker, medium and vessels for cell culturing, positive and negative standard samples, and furthermore, a manual describing how to use the kit.

Compounds selected by a screening method of the present invention are useful as a therapeutic agent for an allergic disease. Alternatively, antisense DNA capable of inhibiting the expression of a marker gene in this invention is useful for this purpose. Such antisense DNAs are those comprising sequences complementary to the sequences comprising preferably at least 20, more preferably 25, 30, 40, 50, and 100 or more continuous nucleotides of the sense strand of a marker gene of this invention. The antisense region may be antisense to any region of transcripts (any transcripts before and after the processing, including the intermediary product) of a marker gene. One example of such region is that comprising the translation initiation codon. Furthermore, an antibody binding to a protein encoded by a marker gene used in the present invention is useful as a therapeutic agent for an allergic disease. One preferred example of the antibody useful as a therapeutic agent in this invention is the one binding to the JAK-interacting domain. A therapeutic agent for an allergic disease according to the present invention comprises a compound selected by the screening method, the antisense DNA, or the antibody as at least one main ingredient, and can be prepared by mixing the ingredient with a physiologically acceptable carrier, excipient, diluent, or such. The therapeutic agent for an allergic disease according to this invention can be administered orally or parenterally to ameliorate allergic symptoms.

For an oral drug, the dosage form can be selected from among granules, powder, tablets, capsules, solution, emulsion, suspension, etc. Examples of injections are subcutaneous, intramuscular, and peritoneal injections.

Furthermore, when a compound to be administered comprises a protein, the therapeutic effect can be achieved by introducing a gene encoding the protein into the living body using a gene therapy technique. A technique for treating a disease by introducing a gene encoding a protein that has a therapeutic effect into the living body to express the protein is well known in the art.

Alternatively, an antisense RNA expression vector comprising the antisense DNA downstream of an appropriate promoter sequence can be administered. When this expression vector is introduced into T cells of an allergic disease patient, the therapeutic effect on allergic disease can be achieved by reducing the expression level of the gene through the expression of the corresponding antisense gene. The expression vector can be introduced into T cells by either known in vivo or ex vivo methods.

Although the dosage may vary depending on the age, sex, body weight, and symptoms of a patient, treatment effects, method for administration, treatment duration, type of the active ingredient comprised in the pharmaceutical composition, and such, the active ingredient can be usually administered in the range of 0.1 mg to 500 mg, preferably 0.5 mg to 20 mg per dose for an adult. However, since the dosage varies according to various conditions, an amount less than the above-described dosage may be sufficient in some cases, and the dosage exceeding the above-described range may be required in other cases. Any prior art literatures cited herein are incorporated by reference.

Brief Description of the Drawings

Fig. 1 is a graph showing the expression levels of SOCS family genes (SOCS1, SOCS3, SOCS5, and CIS1) in T cells of a normal healthy subject and patients with a light, moderate, or severe atopic dermatitis. The vertical axis represents the copy number of each SOCS mRNA per 1 ng RNA of T cells, while the horizontal axis shows test samples.
*: $p<0.05$, **: $p<0.01$
Fig. 2 shows preparation of a dermatitis mouse model and regimen of administration of a prednisolone ointment.
Fig. 3 shows a change in ear edema of the dermatitis mouse model during sensitization with a mite antigen. The upper panel shows the results obtained 14 days after sensitization, and the lower panel shows the results obtained 28 days after sensitization. The vertical axis indicates an ear edema rate (%), and the horizontal axis indicates a sensitization term (weeks) (n = 10, Mean ± S.E.).

$$\text{Ear edema rate (\%)} = (\text{Thickness of ear after sensitization / Thickness of ear before sensitization})$$

Fig. 4 shows the total IgE concentration in the dermatitis mouse model (n = 10, Mean ± S.E.). The vertical axis indicates the total IgE concentration (ng/mL) in blood, and the horizontal axis indicates a sensitization term (weeks). Respective columns correspond, from the left, to the results of a non-sensitized control group, a sensitized control group, and a prednisolone administered group.
Fig. 5 shows expression levels of mRNA of SOCS3 in an auricle of an NC/Nga mite antigen-induced dermatitis mouse model. The vertical axis indicates an expression level of mRNA (copy/ng RNA), and the horizontal axis indicates days after initiation of mite antigen sensitization. Respective columns correspond, from the left, to the results of a non-sensitized control group, a sensitized control group, and a prednisolone administered group.

Best Mode for Carrying out the Invention

The present invention will be explained in more detail below with reference to examples, but is not to be construed as being limited thereto.

[Example 1] Collection of blood samples from patients and normal healthy subjects

To isolate genes that show different expression in an allergic disease-specific manner, blood samples were collected from patients and normal healthy subjects selected after analysis of their symptoms. The blood samples were collected from 10 normal healthy subjects, 7 patients with light atopic dermatitis, 10 patients with moderate atopic dermatitis, and 12 patients with severe atopic dermatitis.

[Example 2] Preparation of lymphocyte fractions from blood samples

T-cells were prepared from a 10 ml blood sample as follows. First, 1 ml of heparin (products of Novo or other manufacturers) was thoroughly spread over the 10 ml-syringe wall surface, and then 10 ml of a blood sample containing a final concentration of 50 units/ml heparin was collected. For blood collection, two 22G needles for each person were prepared. After removing the needle from the syringe, the blood sample was transferred to a 50-ml centrifuge tube (made of polypropylene). The tube was centrifuged at 1500 rpm for 5 min at room temperature and then a 1.1-ml portion was taken from as close to the surface as possible. After further 15, 000 rpm centrifugation for 5 min at 4°C, 1 ml of the supernatant was collected as plasma. An equal amount (9 ml) of 0.9% NaCl containing 3% dextran (Nacalai) was added to the remaining sample. This mixture was inverted gently several times, and then was allowed to stand for 30 min at room temperature.
Platelet rich plasma (PRP) was transferred to a new 15-ml centrifuge tube and centrifuged at 1200 rpm (equivalent to 150 x g for Tomy centrifuge) for 5 min at room temperature. After the centrifugation, platelets were present in the supernatant. Precipitated cells were resuspended in 5 ml Ca and Mg-free HBSS (GIBCO or other manufacturers). The cell suspension was layered on the top of a 5 ml Ficoll Paque (Pharmacia)-containing Falcon tube (2006 or 2059, made of polypropylene) with a Pasteur pipette. Centrifugation was performed at 1200 rpm for 5 min, and at 1500 rpm (equivalent to 400 x g for Tomy centrifuge) for 30 min at room temperature. As a result, granulocytes and erythrocytes were precipitated, lymphocytes, monocytes, and platelets were included in the middle layer, and the Ficoll layer is interposed between the precipitate and the middle layer.

The middle layer was collected using a Pasteur pipette. Two to three volumes of bovine serum albumin (BSA) /phosphate buffered saline (PBS) (0.5% BSA, 2 mM EDTA in PBS, pH 7.2, degassed just before use) were added thereto, and the mixture was centrifuged at 1200 rpm for 5 min at 4°C. The precipitate was collected and washed twice with BSA/PBS solution. After the second wash, cells were resuspended in 5 ml of BSA/PBS, and a portion of the suspension was diluted two-fold with trypan blue to count the cell number. The total cell number was about 1 x $10^7$, and the suspension was used as the lymphocyte fraction. [Example 3] T-cell separation from lymphocyte fraction

The lymphocyte fraction obtained in Example 2 was centrifuged at 1200 rpm for 5 min at 4°C, and the precipitate was resuspended in BSA/PBS at $10^8$ cells/100 µl. The volume was approximately 20 µl. The cell suspension was transferred to an Eppendorf tube (1.5 ml), and then CD3 microbead solution was added thereto. This sample was allowed to stand at 4 to 10°C for 30 min (not on ice) and was then treated using a magnetic cell sorter (MACS, Miltenyi Biotech Inc.) by the following procedure.

An $MS^+/RS^+$ column was set on Mini MACS or Vario MACS separation unit (without needles). BSA/PBS (500 µl) was gently applied onto the column, and the buffer was allowed to flow through the column. Then CD3 microbead-labeled cells were applied onto the column. The column was washed three times with 500 µl BSA/PBS (B-cell fraction). The column was detached from the separation unit and set onto a tube to collect the eluate. BSA/PBS (1 ml) was applied onto the column, and positive cells were eluted rapidly using a plunger attached to the column. The eluate was used as T-cell fraction.

The obtained T-cell fraction was centrifuged at 1200 rpm at 4°C for 5 min. The precipitate was washed twice with BSA/PBS. After the second wash, the cells were resuspended in 1 ml of BSA/PBS, and a portion of the suspension was diluted two-fold with trypan blue to count the cell number. The total cell number was approximately 4 x $10^6$.

[Example 4] Total RNA preparation from T-cells

Total RNA was prepared from T-cells using RNeasy Mini (Qiagen) basically following the manufacturer's instruction. All manipulations were carried out at room temperature, with the manipulator wearing gloves. Four-fold volume of ethanol was added to the wash buffer RPE. To the lysis buffer RLT, 10 µl/ml of 2-mercaptoethanol was added.

The cell suspension was centrifuged at 1000 to 1200 rpm for 5 min, and the supernatant was removed by aspiration. The precipitate was resuspended in 350 µl of lysis buffer RLT (containing 2-mercaptoethanol). At this step, the cell lysate in the lysis buffer RLT was preservable at -70°C. The frozen stored cell lysate was thawn by incubation at 37°C for 10 to 15 min, and, if insoluble matter was observed, was centrifuged for 3 min at maximum speed to collect the supernatant alone. The lysate was homogenized by syringe with a 20G Cathelin needle, and then subjected to QIA shredder; 350 µl of the lysate was applied onto QIA shredder with a Pipetman. The mixture was centrifuged at 1500 rpm for 2 min to collect the eluate. A 350-µl portion of 70% ethanol was added thereto and mixed well by pipetting.

An RNeasy spin column was fixed to the attached 2-ml tube, and the lysate mixture was applied onto the column. The column was centrifuged at 8000 x g (11500 rpm) for 1 min, and the flow through was discarded. Then 700 µl wash buffer RW1 was applied onto the column, and the column was capped and left standing for 5 min. The column was centrifuged at 11500 rpm for 15 seconds, and the flow through was discarded. The column was attached to a new 2-ml tube, 500 µl of wash buffer RPE (with ethanol) was applied onto the column, centrifuged at 11500 rpm for 15 seconds, and the flow through was discarded. Wash buffer RPE (500 µl) was applied onto the column, and centrifuged at a full speed for 2 min. The column was attached to a new tube (1.5 ml), 30 µl of DEPC treated water was applied thereonto, and the capped column was allowed to stand for 10 min. The column was centrifuged at 11500 rpm for 10 min to obtain total RNA. The concentration of the RNA was measured. If the amount was low, the column was set again onto a new 1. 5-ml tube, and 30 µl of DEPC treated water was applied thereonto. Then the column was capped, left standing for 10 min, and centrifuged at 11500 rpm for 10 min to obtain total RNA.

[Example 5] DNase treatment of total RNA

In order to remove DNA from the total RNA prepared from the T-cells, DNase treatment was performed. The treatment was conducted in a reaction mixture containing 2 units of DNase (Nippon Gene) and 50 units of RNase inhibitor (Pharmacia) in 100 µl of 1x DNase buffer (Nippon Gene). After incubating this mixture at 37°C for 15 min, an equal volume of PCI (phenol: chloroform: isoamyl alcohol = 25:24:1) was added thereto, and the tube was vortexed.

The tube was centrifuged at 12000 rpm at room temperature for 10 min, and the upper phase (aqueous phase) was transferred to a new 1. 5-ml tube. One tenth volume of 3 M sodium acetate (pH 5.2), then 2.5 volumes of 100% ethanol and 1 µl of Ethachinmate were added thereto, and the mixture was inverted several times. After allowing the tube to stand at -20°C for 15 min, it was centrifuged at 12000 rpm for 15 min at 4°C. The supernatant was removed, and 70% ethanol was added to the precipitate. After tapping the tube until the precipitate was detached from the tube, the supernatant was completely removed. The precipitate was dried for 3 min and dissolved in 10 to 20 µl of DDW (DNase and RNase free). The concentration was measured, and the sample was stored at -80°C until use.

[Example 6] Quantification of expression amounts using ABI-7700

A part of the total RNA samples prepared from T cells was used for RT-PCR for quantifying the gene expression level by TaqMan method with ABI-PRISM 7700. The TaqMan method is a system for real-time detection and quantification of PCR-amplified DNA strands using fluorescence dyes.

In this method, a primer set prepared based on each SOCS gene nucleotide sequence was used (Table 1). Furthermore, the TaqMan probe was used after labeled with 6-carboxy-fluorescein (FAM) and 6-carboxy-N,N,N',N'-tetramethylrhodamine (TAMRA) at 5'-end and 3'-end, respectively. Nucleotide sequences of the used primer sets and TaqMan probe are shown below.

CIS1 primers
AF025947-f: ACCTGCAGAAGATGCCAGAAG (SEQ ID NO: 3)
AF025947-r: TCGGCATACTCAATGCGTACA (SEQ ID NO: 4)

CIS1 probe
AF025947p: TGTTCACGCTGTCAGTGAAAACCACTCG (SEQ ID NO: 5)

SOCS1 primers
U88326-f: AGACCCCTTCTCACCTCTTGAG (SEQ ID NO: 6)
U88326-r: AGAGGTAGGAGGTGCGAGTTCA (SEQ ID NO: 7)

SOCS1 probe
U88326p: TCCCCCTGGTTGTTGTAGCAGCTTAACT (SEQ ID NO: 8)

SOCS3 primers

AB006967-f: CTTCAGCATCTCTGTCGGAAGA (SEQ ID NO: 9)
AB006967-r: ATCGTACTGGTCCAGGAACTCC (SEQ ID NO: 10)

SOCS3 probe
AB006967p: AACGGCCACCTGGACTCCTATGAGAAAG (SEQ ID NO: 11)

SOCS5 primers

AF073958-f: GGCAGAAGCAGCGTCAGATAT (SEQ ID NO: 12)

AF073958-r: TGTGTGTGGACTTTCCAAGCT (SEQ ID NO: 13)

SOCS5 probe

AF073958p: TGGAGACAGCCATACCCATGTTAGCAGA (SEQ ID NO: 14)

The composition of the reaction solution for monitoring PCR amplification is shown in Table 1. For the standard copy number of each gene, plasmids described below were used. Each plasmid has an insert to be amplified by the above-described primers. Sizes of amplified fragments are set forth in parentheses.

CIS1/pGEM-T easy (120 bp)
SOCS1/pCR2 (120 bp)
SOCS3/pGEM-T easy (102 bp)
SOCSS/pGEM-T easy (76 bp)

Table 1

| Reaction mixture composition for ABI-PRISM 7700 (amount per well) | |
|---|---|
| Sterile distilled water | 25.66 (μl) |
| 10x TaqMan buffer A | 5 |
| 25 mM $MgCl_2$ | 7 |
| dATP (10 mM) | 1.2 |
| dCTP (10 mM) | 1.2 |
| dGTP (10 mM) | 1.2 |
| dUTP (10 mM) | 1.2 |
| Forward Primer (100 μM) | 0.15 |
| Reverse Primer (100 μM) | 0.15 |
| TaqMan Probe (6.7 μM) | 1.49 |
| AmpliTaq Gold (5 U/μl) | 0.25 |
| AmpErase UNG (1 U/μl) | 0.5 |
| Template solution | 5 |
| Total volume | 50 |

The expression amounts of SOCS family genes are shown in Table 2 and Fig. 1.

Table 2

| No. | Pathology | SOCS3 | SOCS5 | SOCS1 | CIS1 |
|---|---|---|---|---|---|
| 1 | Healthy | 1458.08 | 1601.815 | 2329.491 | 3188.494 |
| 2 | Healthy | 351.9105 | 1017.66 | 1406.479 | 2750.802 |
| 3 | Healthy | 968.497 | 3172.139 | 1844.762 | 3096.75 |
| 4 | Healthy | 495.7142 | 628.7065 | 2062.804 | 1227.037 |
| 5 | Healthy | 380.9607 | 703.5109 | 657.1982 | 2736.331 |
| 6 | Healthy | 500.85 | 627.0444 | 1513.615 | 3672.791 |
| 7 | Healthy | 481.6213 | 810.5911 | 659.7459 | 3926.74 |

Table 2 (continued)

| No. | Pathology | SOCS3 | SOCS5 | SOCS1 | CIS1 |
|---|---|---|---|---|---|
| 8 | Healthy | 1249.566 | 1308.318 | 1749.39 | 6959.8 |
| 9 | Healthy | 1788.941 | 1360.944 | 2352.998 | 6168.417 |
| 10 | Healthy | 728.3886 | 1268.932 | 853.7053 | 7641.104 |
| 11 | Light | 807.0501 | 368.1823 | 719.6516 | 1976.83 |
| 12 | Light | 41.45852 | 472.873 | 649.9721 | 884.8137 |
| 13 | Light | 319.5918 | 610.1423 | 1014.481 | 5270.148 |
| 14 | Light | 312.6483 | 791.68 | 627.4481 | 3760.644 |
| 15 | Light | 370.994 | 982.9265 | 631.1635 | 2547.374 |
| 16 | Light | 842.0954 | 1251.164 | 1814.699 | 1835.22 |
| 17 | Light | 1035.245 | 974.4081 | 1124.799 | 5343.038 |
| 18 | Moderate | 1555.719 | 1207.168 | 1692.552 | 3037.704 |
| 19 | Moderate | 4262.705 | 1870.288 | 1788.896 | 7820.949 |
| 20 | Moderate | 783.5549 | 809.9576 | 702.009 | 1160.63 |
| 21 | Moderate | 2895.489 | 1423.61 | 1538.511 | 9594.791 |
| 22 | Moderate | 2418.919 | 2964.095 | 1865.82 | 7750.703 |
| 23 | Moderate | 326.0805 | 713.1234 | 963.3022 | 1835.058 |
| 24 | Moderate | 787.6964 | 799.9439 | 1126.562 | 4280.359 |
| 25 | Moderate | 3129.147 | 983.0927 | 801.9961 | 3274.252 |
| 26 | Moderate | 2037.736 | 681.7354 | 1015.533 | 4577.659 |
| 27 | Moderate | 4112.386 | 1014.482 | 1227.672 | 2573.053 |
| 28 | Severe | 586.8573 | 610.7941 | 488.507 | 2457.098 |
| 29 | Severe | 1304.636 | 1061.391 | 879.1024 | 2663.894 |
| 30 | Severe | 3846.91 | 1503.684 | 1032.858 | 7322.066 |
| 31 | Severe | 1308.956 | 2727.006 | 1299.147 | 6285.055 |
| 32 | Severe | 544.8535 | 328.1689 | 338.7977 | 2238.208 |
| 33 | Severe | 408.0696 | 283.592 | 670.1861 | 2852.863 |
| 34 | Severe | 2886.85 | 1955.84 | 1327.421 | 9790.364 |
| 35 | Severe | 4092.8 | 414.1079 | 638.445 | 3032.142 |
| 36 | Severe | 2847.757 | 1296.949 | 799.8222 | 3102.048 |
| 37 | Severe | 943.9513 | 1017.548 | 1443.823 | 2287.345 |
| 38 | Severe | 2826.906 | 1278.804 | 2537.089 | 8966.379 |
| 39 | Severe | 496.8299 | 1137.852 | 1816.787 | 2102.247 |

As obvious from the above-described results, among the SOCS family genes, only SOCS3 showed significantly high expression levels in test samples from patients. Furthermore, significant differences in the expression level were observed between the healthy and moderate cases, between the healthy and severe cases, furthermore between the light and moderate cases, and the light and severe cases.

[Example 7] Quantitation of mRNA of SOCS3 in auricle of NC/Nga mite antigen-induced dermatitis mouse model

As a model close to human atopic dermatitis, a spontaneous dermatitis model in NC/Nga mice has been reported. A change in an expression level of mRNA of SOCS3 in this animal model was observed.

(1) Preparation of mite antigen-induced dermatitis mouse model using NC/Nga mouse

1. Materials for preparing dermatitis model

As a sensitizing substance for preparing dermatitis, the mite extract-Dp (LSL, Lot No. 756112) was used. The mite extract-Dp is a lyophilized powder extracted from an imago of *Dermatophagoides pteronyssinus* (the family *Pyroglyphidae*). The mite extract-Dp was adjusted to the concentration of 5 mg/ml in water for injection (Otsuka Seiyaku Kogyo, Lot No. 1F76N).

2. Test materials

In order to confirm the effect of existing drugs on the prepared dermatitis model, 0.5% prednisolone ointment (Taisho Pharm. Ind., Ltd., Lot No. P715, P317, PE15) was used.

3. Other drugs

ISOGEN (Wako Pure Chemical Industries, Ltd., Lot No. 78001I, 78001H, 80001I) was used for pre-treatment for RNA extraction and diethyl ether (Wako Pure Chemical Industries, Ltd., Lot No. DMK2137) was used as an anesthetic.

[Animals used]

For a test, 65 NC/Nga male mice, 5 weeks old (Charles River Japan, Inc.) were purchased, and used at 6 weeks old. Mice were reared in a breeding room set at a room temperature of 20 to 26°C (found value 20.0 to 25.1°C), a humidity of 40 to 70% (found value 35.8 to 73.6%), and a lighting time of 12 hours/day (7 to 19 o'clock), and were allowed to freely take a solid feed F-2 (Funabashi farm) and tap water. Mice were pre-reared for 6 days or longer after the arrival, and mice having good general conditions were used for a test.

[Constitution of test groups]

A test was performed using three groups (a non-sensitized control group, a sensitized control group, and a prednisolone administered group). Ten animals from each group were dissected 14 and 28 days after initiation of mite antigen administration.

[Test method]

1. Preparation of dermatitis model

Ten mice per group were used. Five microgram per site of mite antigen (1 μl/site) was intradermally administered to a sensitized control group and a prednisolone administered group in left and right auricles and two places of a back, hair of which had been cut in advance, (total four places) at 3-day intervals (total 5 times administration in the case of dissection 14 days after initiation of mite antigen administration, total 9 times administration in the case of dissection 28 days after initiation of mite antigen administration). Regimen for preparing a dermatitis model in this Example is shown in Fig. 2.

2. Administration of prednisolone ointment

A prednisolone ointment was transdermally administered to animals of a prednisolone administered group starting the 9th day after initiation of mite antigen administration. A dose of a prednisolone ointment was 30 mg per each mite antigen administration site (30 mg x 4 places, 120 mg/animal/time). Administration regimen is shown in Fig. 2.

3. Measurement of ear edema

Thickness of left and right ears were measured using a dial thickness gauge once a week for all groups during the test term. Measurement regimen is shown in Fig. 2.

4. Observation of symptoms of back skin (mite antigen administration site)

Symptoms of a mite antigen administration site were observed once a week for all groups during the test term. Observation was scored with reference to an assessment standard of clinical symptoms of human atopic dermatitis. Observation regimen is shown in Fig. 2.

Assessment items: (i) pruritus, (ii) flare/bleeding, (iii) edema, (iv) abrasion/tissue deficiency, (v) drying
Score: 0 = asymptomatic, 1 = mild, 2 = moderate, 3 = severe

The respective items were scored, and a sum of each score was regarded as a score of an individual.

5. Measurement of blood total IgE concentration

Animals for dissection 28 days after mite antigen sensitization was bled before mite antigen administration, and 14 days (bled from orbital vein) and 28 days (bled from abdominal cava) after initiation of administration. The blood samples were centrifuged, and serum was stored at -80°C. The blood total IgE concentration in the stored serum was measured using a mouse IgE measuring kit (Yamasa EIA, Lot No. P102: Yamasa Corporation). Measurement regimen is shown in Fig. 2.

6. Pathohistological test

Animals were sacrificed (by exsanguination under ether anesthesia) 14 days after initiation of mite antigen administration, and mite antigen administration sites (left and right auricles and two places of back skin) , thymus, spleen, and lymph node were removed. One piece each of auricles and back skin, and thymus, spleen, and lymph node were used for extracting RNA. After the remaining auricle and skin (one piece each) were fixed with a 10% neutral buffered formalin solution, a paraffin block was prepared according to the conventional method, and subjected to hematoxylin eosin staining, toluidine blue staining, and Congo red staining, and observed under an optical microscope. Test regimen is shown in Fig. 2.

7. Pre-treatment before RNA extraction

One piece each (about 1 square centimeter) of auricles and back skin, and thymus, spleen, and lymph node removed were placed into a 15-mL Falcon tube. Then, 5 mL of ISOGEN was poured therein, and homogenized under ice-cooling using a homogenizer (NS-310E; Nichionirikakikai Seisakusho). After completion of homogenization, the samples were immersed into liquid nitrogen and frozen. The samples were stored at -80°C until RNA extraction.

[Test results]

1. Measurement of ear edema

The results of measurement of a change in ear edema of dermatitis model mice during mite antigen sensitization are shown in Fig. 3. Both 14 days after mite antigen administration (Fig. 3 upper) and 28 days after mite antigen administration (Fig. 3 lower) , no ear edema was perceived in the non-sensitized control group. The sensitized control group showed clear tylosis of an ear from one week after mite antigen administration, and a high score value continued after two weeks later. The prednisolone administered group showed clear tylosis of an ear from one week after mite antigen administration, and an increase in ear edema was inhibited from the second week.

2. Observation of symptoms of back skin

Both 14 days after mite antigen administration and 28 days after mite antigen administration, individuals having the alleviated symptoms of dermatitis appeared from two weeks after mite antigen administration.

3. Measurement of blood total IgE concentration

Fig. 4 shows the total IgE concentration. In the sensitized control group and the prednisolone administered group, to which blood mite antigen had been administered, the blood total IgE concentration was increased from two weeks after mite antigen administration.

4. Pathohistological test of auricle skin

<u>Dissection 14 days after mite antigen administration</u>

One out of 10 cases in the non-sensitized control group showed tylosis of epidermis as well as inflammatory cellular infiltration (mainly lymphocytes) and increase in edema and connective tissues in dermis and subcutaneous tissues, but these symptoms were all mild. No abnormal observation was perceived in the remaining 9 out of 10 cases. The sensitized control group showed tylosis of epidermis, as well as inflammatory cellular infiltration (neutrophils, eosinophils, histocytes, and lymphocytes, etc.), increase in edema and connective tissues, and degranulation of mast cells in dermis and subcutaneous tissues; a degree of these symptoms were all mild to moderate. Six out of ten cases also showed ulcer formation.

The prednisolone administered group presented tylosis of epidermis, as well as inflammatory cellular infiltration (neutrophils, eosinophils, histocytes, and lymphocytes, etc.), increase in edema and connective tissue, and degranulation of mast cells in dermis and subcutaneous tissues; a degree of these symptoms were all mild to moderate. Six out of ten cases also presented ulcer formation.

Dissection 28 days after mite antigen administration

In the non-sensitized control group, no abnormal observation was perceived in epidermis, dermis, and subcutaneous tissues. The sensitized control group showed tylosis of epidermis, and also inflammatory cellular infiltration (neutrophils, eosinophils, histocytes, and lymphocytes, etc.), increase in edema and connective tissues, and degranulation of mast cells and the like symptoms in dermis and subcutaneous tissues; a degree of these symptoms were all mild to moderate. Nine out of ten cases also presented ulcer formation.

The prednisolone administered group presented tylosis of epidermis, as well as inflammatory cellular infiltration (neutrophils, eosinophils, histocytes, and lymphocytes, etc.), increase in edema and connective tissues, and degranulation of mast cells and the like symptoms in dermis and subcutaneous tissues; these symptoms were all mild to moderate and accompanied with ulcer formation.

5. Pathohistological test of back skin

Dissection 14 days after mite antigen administration

In the non-sensitized control group, no abnormal observation was perceived. The sensitized control group showed tylosis of epidermis, as well as inflammatory cellular infiltration (neutrophils, eosinophils, histocytes, and lymphocytes, etc.), increase in edema and connective tissues, and degranulation of mast cells in dermis and subcutaneous tissues. Three out of ten cases also showed ulcer formation.

The prednisolone administered group showed tylosis of epidermis, as well as inflammatory cellular infiltration (neutrophils, eosinophils, histocytes, and lymphocytes, etc.), increase in edema and connective tissues, and degranulation of mast cells and the like symptoms in dermis and subcutaneous tissues. In addition, ulcer formation was accompanied in one out of ten cases.

Dissection 28 days after mite antigen administration

In the non-sensitized control group, no abnormal observation was perceived. The sensitized control group showed the same degree of symptoms as those in the cases obtained 14 days after mite antigen administration.

The prednisolone administered group showed the same degree of symptoms as those in the cases obtained 14 days after mite antigen administration.

(2) Quantitation of SOCS3 mRNA in auricles of NC/Nga mite antigen-induced dermatitis mouse model

SOCS3 mRNA in the NC/Nga mouse model prepared in the above (1) was quantitated. Auricles of mice dissected 14 days and 28 days after mite antigen administration were frozen with ISOGEN to serve as samples.

Synthesis of cDNA from auricle tissues

The ISOGEN-frozen samples were thawed on ice, and homogenized with a 20G Cathelin injection needle (Terumo Corp.). A 600-μL portion of 5 mL of the resulting homogenate was taken into a 1.5-mL tube, and total RNA was extracted according to the protocol for the ISOGEN product. The extracted RNA was subjected to RNeasy column (QUIAGEN) treatment according to the product protocol. Then, DNase (RT grade, Nippon Gene Co., Ltd.) treatment was performed according to the product protocol. From 1 μg of the resulting total RNA, cDNA was synthesized using Random primers (GIBCO BRL) and Super script II (Invitrogen) according to a product protocol, then treated with RNase H.

Quantitation of mRNA

The same concentration of cDNAs of 10 animals per each mouse test group were mixed. The mixed cDNA (5 ng) was subjected to quantitation of mouse SOCS3 mRNA using ABI7700. The quantitated value was corrected using the quantitated value of mouse β-actin as an internal standard. Nucleotide sequences of primers and probes used in this quantitation experiment are shown below.

Mouse SOCS3 (accession No. U88328)
Forward primer (SEQ ID NO: 15): 5'-CTTTCTTATCCgCgACAgCTC-3'
Reverse primer (SEQ ID NO: 16): 5'-CACTggATgCgTAggTTCTTg-3'
Probe (SEQ ID NO: 17): 5'-FAM-CACTggATgCgTAggTTCTTg-TAMRA-3'

Mouse β-actin (accession No. X03672)
Forward primer (SEQ ID NO: 18): 5'-ACTATTGGCAACGAGCGGTTC-3'
Reverse primer (SEQ ID NO: 19): 5'-GGATGCCACAGGATTCCATACC-3'
Probe (SEQ ID NO: 20): 5'-FAM-CCTGAGGCTCTTTTCCAGCCTTCCTTCT-TAMRA-3'

Fig. 5 shows the results of quantitation of SOCS3 mRNA expression in auricles of the NC/Nga mite antigen-induced dermatitis mouse model. Auricles of the NC/Nga mite antigen-induced dermatitis mouse model expressed SOCS3 mRNA at high levels in the sensitized control group and the prednisolone administered group as compared with the non-sensitized control group at both 14 days and 28 days after initiation of mite antigen stimulation. Although prednisolone coating did not clearly change the expression level, the blood total IgE concentration in the prednisolone administered group was high similar to that in the sensitized group. Furthermore, the pathohistological finding revealed that prednisolone coating did not improve the mite antigen-induced dermatitis symptoms, indicating that SOCS3 mRNA expression reflects dermatitis symptoms.

From the foregoing results, it can be considered that significantly high SOCS3 mRNA expression in human peripheral blood T cells in a dermatitis patient was also confirmed in mice dermatitis models.

Industrial Applicability

The present invention provided SOCS3 gene whose expression level significantly increases in T cells of atopic dermatitis patients. Using marker genes of this invention, it was possible to test for an allergic disease and screen for a therapeutic agent for the disease.

Expression levels of allergic disease-associated genes provided by the present invention can be easily detected regardless of the types of allergens. Therefore, pathological conditions of allergic diseases can be comprehensively understood.

In addition, using peripheral blood mononuclear cells as a sample, the expression level of the genes can be analyzed in a much less invasive manner to patients according to methods of testing for an allergic disease of the present invention. Furthermore, gene expression analysis methods of the present invention, in contrast to protein measurements such as ECP, enable highly sensitive measurement using a trace sample. Gene analysis technique trends toward high-throughput and lower prices. Therefore, test methods according to the present invention are expected to become important bedside diagnostic methods in the near future. In this sense, the diagnostic value of the gene associated with pathological conditions is high.

**Claims**

**1.** A method of testing for an allergic disease, which comprises the steps of:

(a) measuring the expression level of a marker gene in a biological sample from a test subject;
(b) comparing the expression level with that of the marker gene in a biological sample from a healthy subject;

and
(c) judging the test subject as being affected with an allergic disease when the expression level of the marker gene in a biological sample from the test subject is found to be significantly elevated,

wherein the marker gene is an SOCS3 gene.

**2.** The testing method according to claim 1, wherein the allergic disease is atopic dermatitis.

**3.** The testing method according to claim 1, wherein the gene expression level is measured by using cDNA PCR.

**4.** The testing method according to claim 1, wherein the gene expression level is measured by detecting a protein encoded by the gene.

**5.** The testing method according to claim 1, wherein the biological sample comprises peripheral blood T cells.

**6.** A reagent for testing for an allergic disease, which comprises an oligonucleotide comprising at least 15 nucleotides complementary to a polynucleotide which comprises a nucleotide sequence of an SOCS3 gene or the complementary strand thereof.

**7.** A reagent for testing for an allergic disease, which comprises an antibody which recognizes a peptide comprising an amino acid sequence encoded by an SOCS3 gene.

**8.** A method for screening for a therapeutic agent for an allergic disease, which comprises the steps of:

(1) contacting a candidate compound with a cell expressing a marker gene;
(2) measuring the expression level of the marker gene; and
(3) selecting a compound which decreases the expression level of the marker gene compared to a control in which the candidate compound has not been contacted,

wherein the marker gene is an SOCS3 gene or a gene functionally equivalent thereto.

**9.** The method according to claim 8, wherein the cell is T cell.

**10.** A method of screening for a therapeutic agent for an allergic disease, which comprises the steps of:

(1) administering a candidate compound to a test animal;
(2) measuring the expression level of a marker gene in a biological sample from the test animal; and
(3) selecting a compound which decreases the expression level of the marker gene compared to a control in which the candidate compound has not been administered,

wherein the marker gene is an SOCS3 gene or a gene functionally equivalent thereto.

**11.** A method of screening for a therapeutic agent for an allergic disease, which comprises the steps of:

(1) contacting a candidate compound with cells carrying a vector comprising a transcriptional control region of a marker gene and a reporter gene that is expressed under the control of the transcriptional control region;
(2) measuring the activity of the reporter gene; and
(3) selecting a compound which decreases the expression level of the reporter gene compared to a control in which the candidate compound has not been contacted,

wherein the marker gene is an SOCS3 gene or a gene functionally equivalent thereto.

**12.** A method of screening for a therapeutic agent for an allergic disease, which comprises the steps of:

(1) contacting a candidate compound with a protein encoded by a marker gene;
(2) measuring the activity of the protein; and
(3) selecting a compound which decreases the activity of the protein compared to a control in which the candidate compound has not been contacted,

wherein the marker gene is an SOCS3 gene or a gene functionally equivalent thereto.

**13.** A therapeutic agent for an allergic disease, which comprises as an active ingredient a compound obtainable by the screening method according to any one of claims 8, 10, 11, and 12.

**14.** A therapeutic agent for an allergic disease, which comprises as a main ingredient an antisense DNA that contains a sequence complementary to a sequence comprising at least 15 continuous nucleotides of the sense strand sequence of a marker gene, wherein the marker gene is an SOCS3 gene or a gene functionally equivalent thereto.

**15.** A therapeutic agent for an allergic disease, which comprises as a main ingredient an antibody which binds to a protein encoded by a marker gene, wherein the marker gene is an SOCS3 gene or a gene functionally equivalent thereto.

**16.** Use of a transgenic nonhuman vertebrate, in which the expression level of a marker gene has been increased in T cells, as an allergic disease animal model, wherein the marker gene is an SOCS3 gene or a gene functionally equivalent thereto.

**17.** A kit for screening for a therapeutic agent for an allergic disease, the kit comprising an oligonucleotide comprising at least 15 nucleotides complementary to a polynucleotide comprising the nucleotide sequence of a marker gene or the complementary strand thereof and cells expressing the marker gene, wherein the marker gene is an SOCS3 gene or a gene functionally equivalent thereto.

**18.** A kit for screening for a therapeutic agent for an allergic disease, the kit comprising an antibody which recognizes a peptide comprising an amino acid sequence encoded by a marker gene and cells expressing the marker gene, wherein the marker gene is an SOCS3 gene or a gene functionally equivalent thereto.

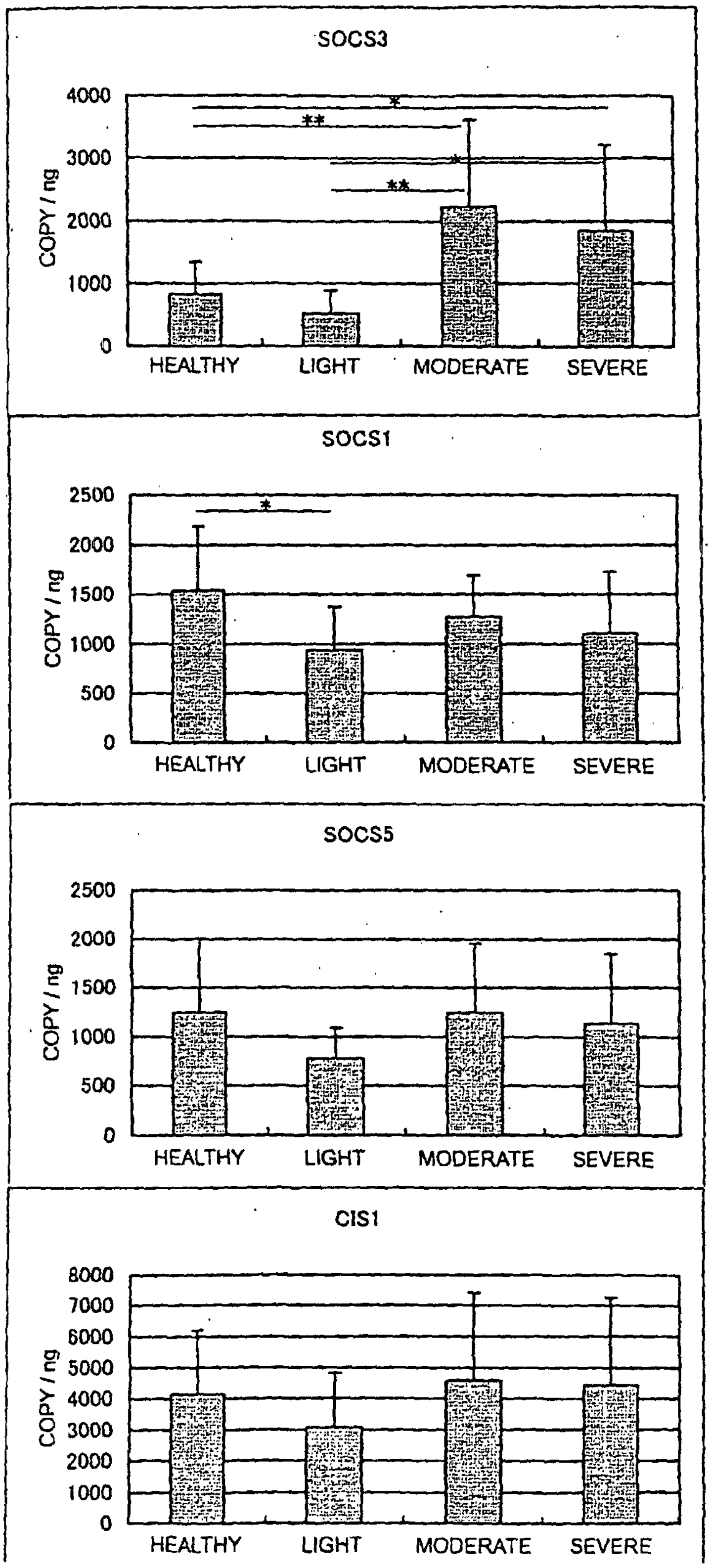
SOCS3
4000
3000
2000
1000
0
COPY / ng
*
**
*
**
HEALTHY
LIGHT
MODERATE
SEVERE
SOCS1
2500
2000
1500
1000
500
0
COPY / ng
*
HEALTHY
LIGHT
MODERATE
SEVERE
SOCS5
2500
2000
1500
1000
500
0
COPY / ng
HEALTHY
LIGHT
MODERATE
SEVERE
CIS1
8000
7000
6000
5000
4000
3000
2000
1000
0
COPY / ng
HEALTHY
LIGHT
MODERATE
SEVERE

FIG. 1

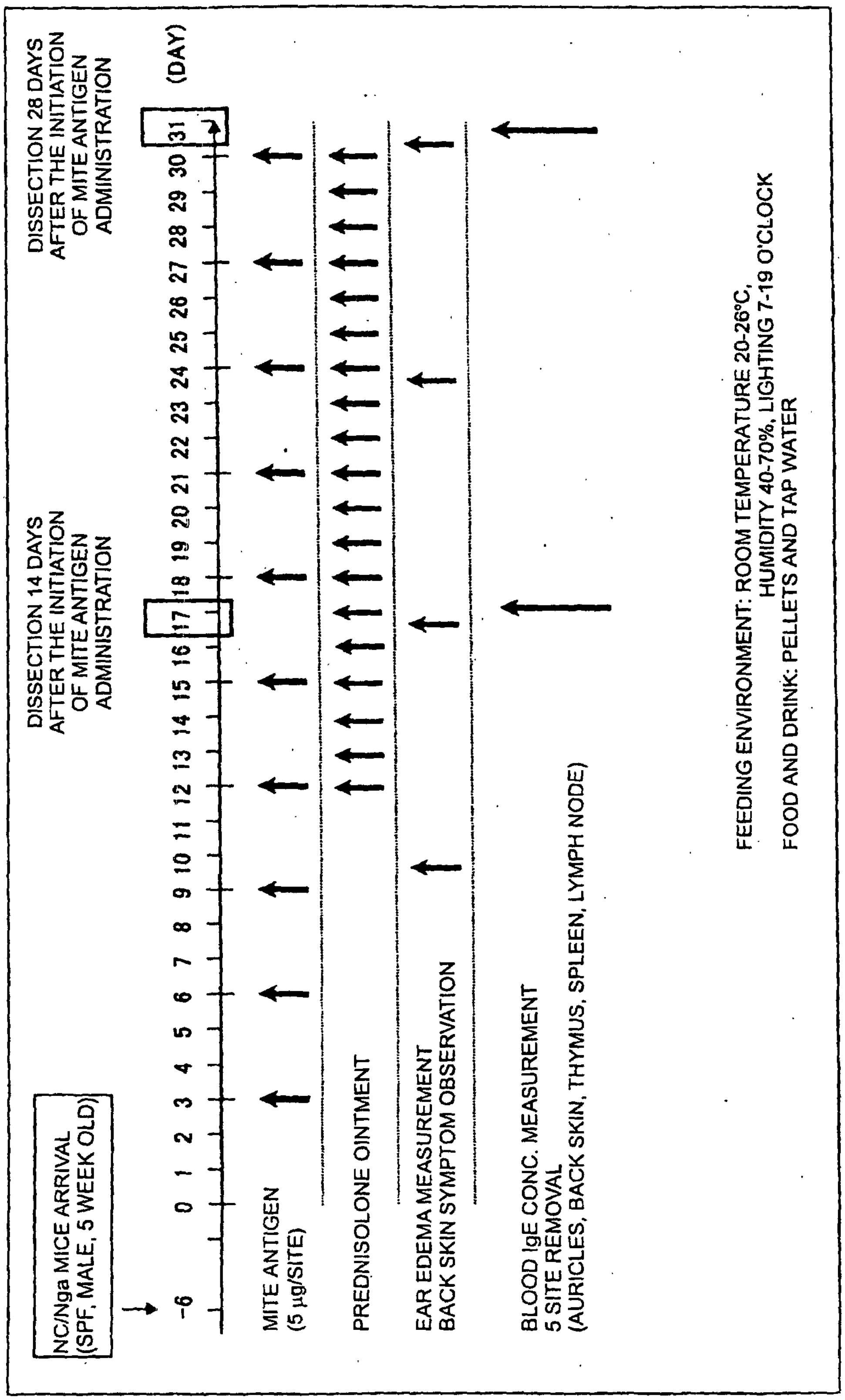
NC/Nga MICE ARRIVAL
(SPF, MALE, 5 WEEK OLD)
DISSECTION 14 DAYS
AFTER THE INITIATION
OF MITE ANTIGEN
ADMINISTRATION
DISSECTION 28 DAYS
AFTER THE INITIATION
OF MITE ANTIGEN
ADMINISTRATION
-6 0 1 2 3 4 5 6 7 8 9 10 11 12 13 14 15 16 17 18 19 20 21 22 23 24 25 26 27 28 29 30 31 (DAY)
MITE ANTIGEN
(5 μg/SITE)
PREDNISOLONE OINTMENT
EAR EDEMA MEASUREMENT
BACK SKIN SYMPTOM OBSERVATION
BLOOD IgE CONC. MEASUREMENT
5 SITE REMOVAL
(AURICLES, BACK SKIN, THYMUS, SPLEEN, LYMPH NODE)
FEEDING ENVIRONMENT: ROOM TEMPERATURE 20-26°C,
HUMIDITY 40-70%, LIGHTING 7-19 O'CLOCK
FOOD AND DRINK: PELLETS AND TAP WATER

FIG. 2

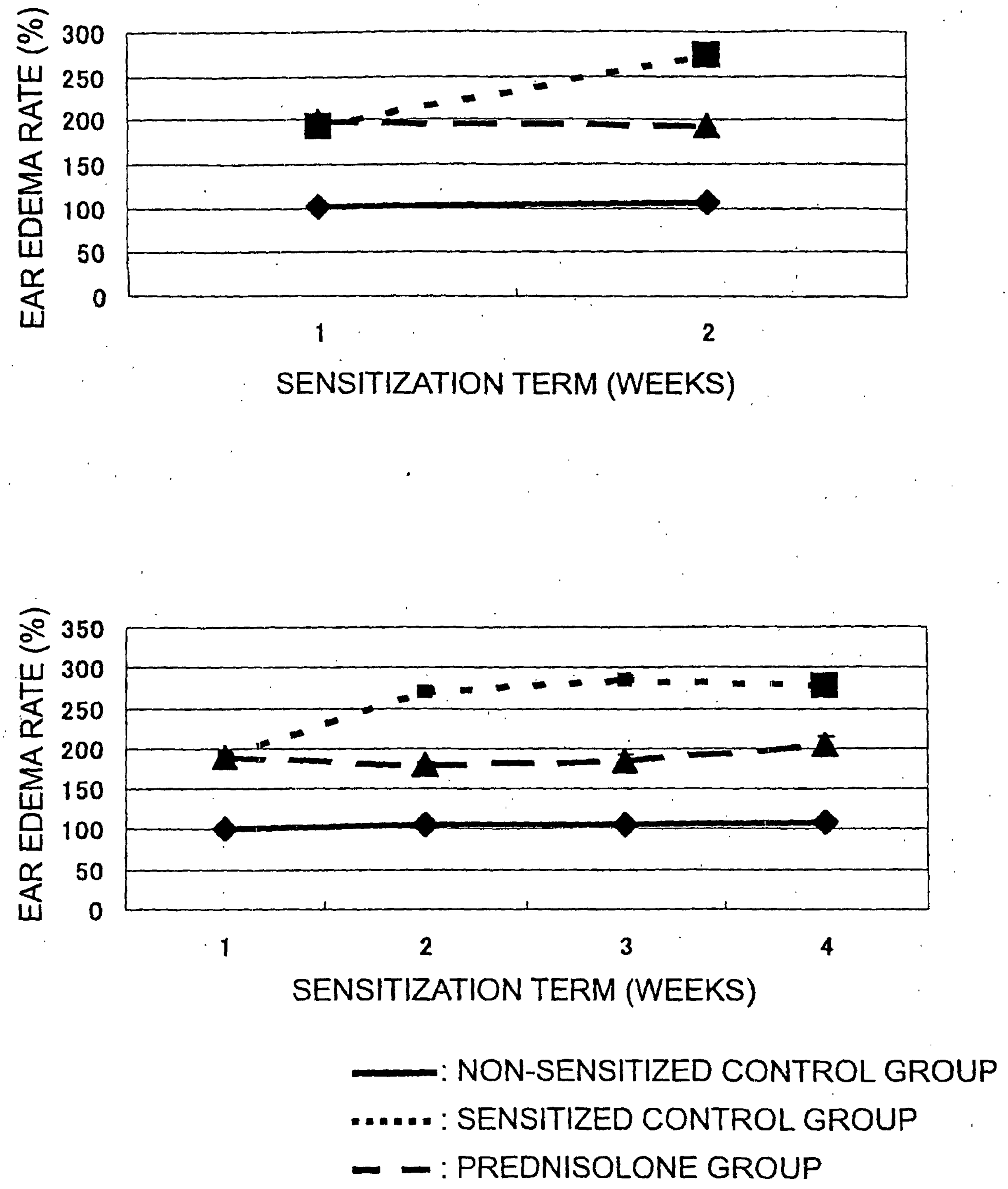
EAR EDEMA RATE (%)
300
250
200
150
100
50
0
1
2
SENSITIZATION TERM (WEEKS)
EAR EDEMA RATE (%)
350
300
250
200
150
100
50
0
1
2
3
4
SENSITIZATION TERM (WEEKS)
: NON-SENSITIZED CONTROL GROUP
: SENSITIZED CONTROL GROUP
: PREDNISOLONE GROUP

FIG. 3

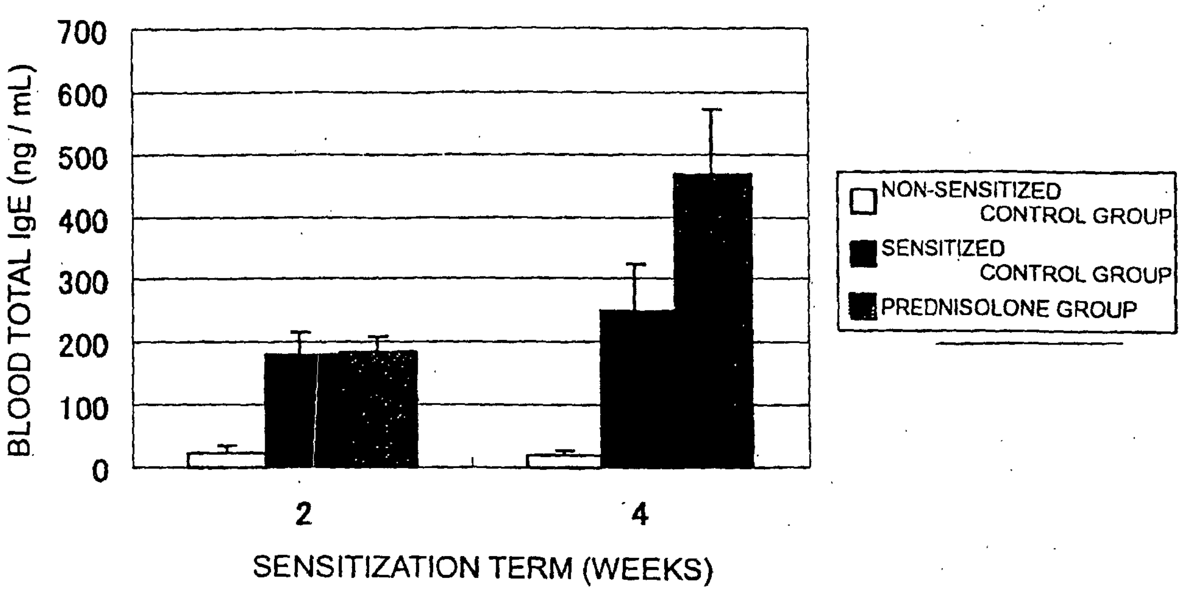
700
600
500
400
300
200
100
0
BLOOD TOTAL IgE (ng / mL)
2
4
SENSITIZATION TERM (WEEKS)
NON-SENSITIZED CONTROL GROUP
SENSITIZED CONTROL GROUP
PREDNISOLONE GROUP

FIG. 4

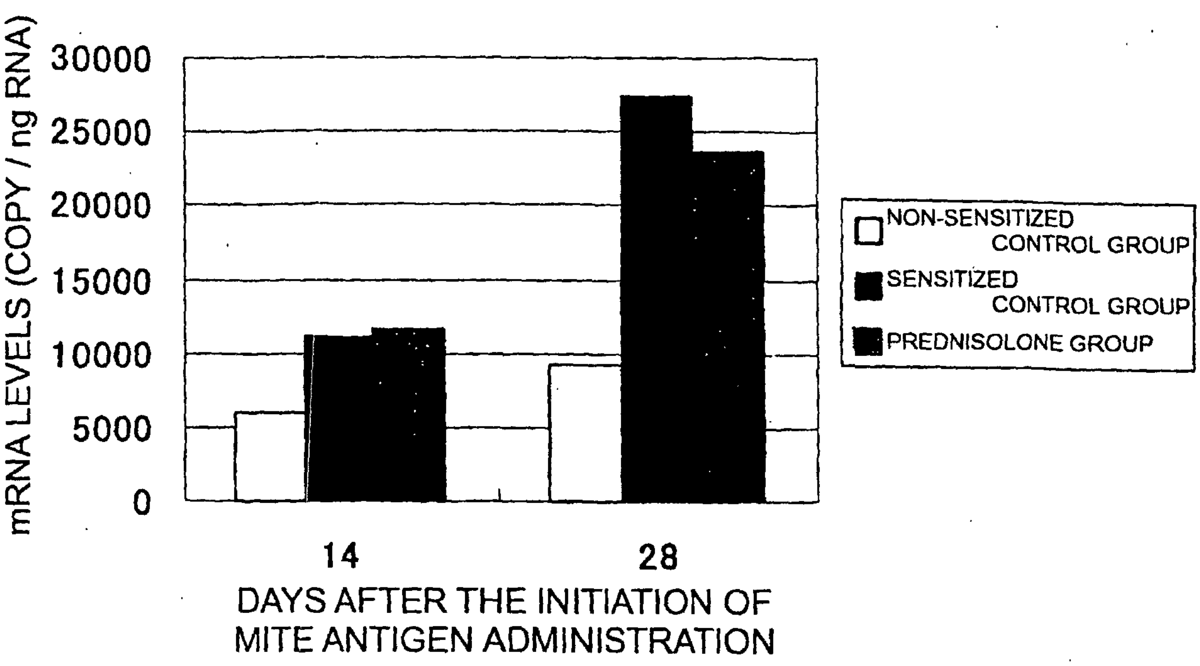
mRNA LEVELS (COPY / ng RNA)
30000
25000
20000
15000
10000
5000
0
14
28
DAYS AFTER THE INITIATION OF
MITE ANTIGEN ADMINISTRATION
NON-SENSITIZED CONTROL GROUP
SENSITIZED CONTROL GROUP
PREDNISOLONE GROUP

FIG. 5

## INTERNATIONAL SEARCH REPORT

International application No.
PCT/JP03/00600

A. CLASSIFICATION OF SUBJECT MATTER
Int.Cl[7] C12N15/09, C12Q1/68, G01N33/15, G01N33/50

According to International Patent Classification (IPC) or to both national classification and IPC

B. FIELDS SEARCHED

Minimum documentation searched (classification system followed by classification symbols)
Int.Cl[7] C12N15/09, C12Q1/68, G01N33/15, G01N33/50

Documentation searched other than minimum documentation to the extent that such documents are included in the fields searched

Electronic data base consulted during the international search (name of data base and, where practicable, search terms used)
MEDLINE(STN), WPI(DIALOG), BIOSIS(DIALOG), JICST FILE(JOIS), GenBank/EMBL/DDBJ/GeneSeq, SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| P,X | FEDERICI, M. et al., Impaired IFN-gamma-dependent Inflammatory Responses in Human Keratinocytes Overexpressing the Suppressor of Cytokine Signaling 1. J.Immunol. (June, 2002), Vol.169, No.1, pages 434 to 442 | 6-12,14-18 |
| P,X | HONG, F. et al., Opposing roles of STAT1 and STAT3 in T cell-mediated hepatitis: regulation by SOCS. J.Clin.Invest. (November, 2002), Vol.110, No.10, pages 1503 to 1513 | 6-12,14-18 |
| P,X | Mitsuhito KUBO, "SOCS Bunshi ni yoru Th1/Th2 Bunka Seigyo to Allergy tono Kankei", Protein, Nucleic acid and Enzyme, 10 December, 2002 (10.12.02), Vol.47, No.16, pages 2279 to 2285 | 6-12,14-18 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 March, 2003 (20.03.03) | 25 March, 2003 (25.03.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/00600

**Box I Observations where certain claims were found unsearchable (Continuation of item 2 of first sheet)**

This international search report has not been established in respect of certain claims under Article 17(2)(a) for the following reasons:

1. [X] Claims Nos.: 1-5
because they relate to subject matter not required to be searched by this Authority, namely:
The inventions as set forth in the above claims involve methods for treatment of the human body by therapy, as well as diagnostic methods, and thus relate to a subject matter which this International Searching Authority is not required, under the provisions of Article (continued to extra sheet)

2. [X] Claims Nos.: 13
because they relate to parts of the international application that do not comply with the prescribed requirements to such an extent that no meaningful international search can be carried out, specifically:
Concerning "a compound which can be obtained by a screening method as set forth in any of claims 8, 10, 11 and 12" as set forth in the above claim, it is completely unknown what specific compounds are involved in the scope thereof and what are not. Thus the above claim is (continued to extra sheet)

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

## INTERNATIONAL SEARCH REPORT

International application No.
PCT/JP03/00600

C (Continuation). DOCUMENTS CONSIDERED TO BE RELEVANT

| Category* | Citation of document, with indication, where appropriate, of the relevant passages | Relevant to claim No. |
|---|---|---|
| X | MAIER, J. et al., Regulation of Signal Transducer and Activator of Transcription and Suppressor of Cytokine-Signaling Gene Expression in the Brain of Mice with Astrocyte-Targeted Production of Interleukin-12 or Experimental Autoimmune Encephalomyelitis. Am.J.Pathol. (January, 2002), Vol.160, No.1, pages 271 to 288 | 6-12,14-18 |
| X | SHOUDA, T. et al., Induction of the cytokine signal regulator SOCS3/CIS3 as a therapeutic strategy for treating inflammatory arthritis. J.Clin.Invest. (2001), Vol.108, No.12, pages 1781 to 1788 | 6-12,14-18 |
| X | WO 01/29178 A2 (SMITHKLINE BEECHAM CORP.), 26 April, 2001 (26.04.01), Full text & EP 1224220 A2 | 6-12,14-18 |
| A | WO 98/20023 A1 (HALL INST MEDICAL RES WALTER & ELIZA), 14 May, 1998 (14.05.98), Full text & AU 9746943 A & GB 2331753 A & NO 9902116 A & EP 948522 A1 & CN 1253565 A & JP 2001-502183 A & KR 2000053017 A & US 2002/0147307 A1 | 6-12,14-18 |
| A | WO 99/23220 A1 (INCYTE PHARM INC.), 14 May, 1999 (14.05.99), Full text & AU 9912055 A & EP 970215 A1 & JP 2001-508312 A | 6-12,14-18 |
| A | WO 00/63357 A2 (BETH ISRAEL DEACONESS MEDICAL CENT), 26 October, 2000 (26.10.00), Full text & AU 200046519 A & US 2002/0142466 A1 | 6-12,14-18 |
| A | WO 01/79555 A2 (MILLENNIUM PHARM INC.), 25 October, 2001 (25.10.01), Full text & AU 200151612 A | 6-12,14-18 |

Form PCT/ISA/210 (continuation of second sheet) (July 1998)

**INTERNATIONAL SEARCH REPORT**

International application No.
PCT/JP03/00600

Continuation of Box No.I-1 of continuation of first sheet(1)

17(2)(a)(i) of the PCT and Rule 39.1(iv) of the Regulations under the PCT, to search.

Continuation of Box No.I-2 of continuation of first sheet(1)

described in an extremely unclear manner. Such being the case, no meaningful international search can be made on the above claim.

Form PCT/ISA/210 (extra sheet) (July 1998)